# EUROPEAN PATENT SPECIFICATION

(11) **EP 1 630 228 B1**
(45) Date of publication and mention of the grant of the patent: **11.01.2012**
(21) Application number: 05018480.3
(22) Date of filing: 25.08.2005
(51) Int. Cl.: C12N 15/10

(54) **Method for separation and purification of nucleic acid**
Verfahren zur Trennung und Reinigung von Nukleinsäure
Procédé de séparation et purification d'acide nucléique

(30) Priority: 25.08.2004 JP 2004244750; 09.02.2005 JP 2005032986
(43) Date of publication of application: 01.03.2006
(73) Proprietor: FUJIFILM Corporation, Minato-ku Tokyo (JP)
(72) Inventor: Komazawa, Hiroyuki, Fuji Photo Film Co., Ltd, Asaka-shi Saitama (JP); Momoki, Yasuhito, Fuji Photo Film Co., Ltd, Asaka-shi Saitama (JP); Hando, Rie, Fuji Photo Film Co., Ltd, Asaka-shi Saitama (JP); Iwaki, Yoshihide, Fuji Photo Film Co., Ltd, Asaka-shi Saitama (JP)
(74) Representative: Hiebl, Inge Elisabeth

(56) References cited:
- EP-A- 1 382 675
- EP-A- 1 382 677
- SCOTT J F ET AL: "ISOLATION OF RNA FROM ANIMAL TISSUE BY USE OF CHAOTROPIC AGENTS" ANALYTICAL BIOCHEMISTRY, vol. 47, no. 2, 1972, pages 471-480, XP009059340 ISSN: 0003-2697

## Description

### Technical Field

The present invention relates to a method for separation and purification of nucleic acid, particularly in animal tissues and also to a solid phase and a treating solution used for the aforementioned method.

### Background Art

Nucleic acid is used in various forms in various fields. For example, in the field of recombinant nucleic acid techniques, nucleic acid is demanded to be used in forms of genomic nucleic acid, nucleic acid probe and plasmid nucleic acid.

Nucleic acid is used in various methods in the field of diagnosis as well. For example, nucleic acid probe is routinely used for detection and diagnosis of pathogenic organisms for human being. Similarly, nucleic acid is used for detection of Mendelian disorder. Nucleic acid is also used for detection of polluting substances for food. Further, due to various reasons from preparation of genetic map to cloning and recombination expression, nucleic acid is routinely used in confirmation of position, identification and isolation of interesting nucleic acid.

In many cases, nucleic acid is available only in very small amounts and an operation for isolation and purification is troublesome and time-consuming. Such a troublesome operation which is often time-consuming is apt to result in loss of nucleic acid. In purification of nucleic acid prepared from animal tissues, blood, urine and bacterial culture, a risk of causing contamination is also resulted.

As one of the methods for simple and efficient separation and purification of nucleic acid, there has been reported a method where a solution for adsorption of nucleic acid with a solid phase and a solution for desorption of nucleic acid from a solid phase are used so that nucleic acid is adsorbed with and desorbed from, respectively, a solid phase comprising an organic macromolecule having a hydroxyl group on its surface whereby nucleic acid is separated and purified (Japanese Patent Laid-Open No. 2003/128,691).

EP 1 382 675 discloses a method for separating and purifying a nucleic acid comprising a step of adsorbing and desorbing a nucleic acid to and from a membrane of an organic macromolecule which has a membrane thickness of 10 µm to 500 µm.

EP 1 382 677 discloses a method for separating and purifying RNA from a nucleic acid mixture comprising a step of adsorbing and desorbing a nucleic acid in the nucleic acid mixture containing RNA and DNA to and from a solid phase of an organic macromolecule.

Jesse F. Scott et al., Analytical Biochemistry 47, 471-480 (1972), discloses a method for isolating of ribonucleic acid from animal tissue, using Li(TCA)² as chaotropic agent for the extraction of nucleic acids from whole cells, isolated nuclei, and the insoluble material which collects at the interface between the aqueous and organic phases during phenol extraction procedures.

### DISCLOSURE OF THE INVENTION

When the aforementioned method for separation and purification of nucleic acid is carried out and particularly when nucleic acid is separated and purified hom a solution prepared by treatment of animal tissue, it is requested to recover the aimed nucleic acid from small animal tissues quickly, in high yield and in high purity. An object of the present invention is to provide a method for preparing nucleic acid quickly, in high yield and in high purity in a method where nucleic acid in a solution containing nucleic acid prepared from animal tissues is adsorbed with the surface of a solid phase, washed, etc. and desorbed to separate and purity the nucleic acid.

In order to solve the aforementioned problems, the present inventors have conducted intensive investigations and found that, when a solid phase comprising an organic macromolecule having a polysaccharide structure is used as a solid phase in adsorption of nucleic acid in a nucleic acid-containing solution, particularly prepared from animal tissues, the aimed nucleic acid is able to be recovered quickly, in high yield and in high degree of purification. It has been found that, in the present invention, yield and purity of the aimed nucleic acid are dramatically improved particularly when a solid phase comprising an organic macromolecule prepared by saponification of acetylcellulose or of a mixture of acetylcelluloses having different acetyl values is used. The present invention has been achieved on the basis of those findings.

Thus, in accordance with the present invention, a method for separation and purification of nucleic acid comprising a step of adsorbing to and desorbed nucleic acid from a solid phase using a solution containing nucleic acid, particularly prepared from animal tissues, and a solution desorbing nucleic acid from the solid phase, respectively, wherein a solid phase comprises an organic macromolecule having a polysaccharide structure or, preferably, a solid phase comprising an organic macromolecule prepared by saponification of acetyl cellulose or a mixture of acetyl celluloses having different acetyl values.

In the present invention, the aforementioned objects are achieved by the following constitutions.
1. A method for separation and purification of nucleic acid comprising:
   (1) adding a pretreatment solution and further adding a water-soluble organic solvent to a sample solution containing nucleic acid to prepare a solution containing nucleic acid for adsorbing to a solid phase;
   (2) passing the solution containing nucleic acid for adsorbing to a solid phase through a solid phase, to adsorb nucleic acid to the solid phase;
   (3) passing a washing solution through the solid phase, to wash the solid phase under such a state that nucleic acid is adsorbed; and
   (4) passing an elution solution through the solid phase to desorb nucleic acid from the solid phase,
   wherein the pretreatment solution contains BisTris (bis(2-hydroxyethyl)iminotris(hydroxymethyl)methane) as a buffer and is a solution containing a surfactant and at least one selected from the group consisting of antifoaming agent, stabilizer for nucleic acid and chaotropic salt, and the solid phase is a solid phase comprising an organic macromolecule having a polysaccharide structure.
2. The method for separation and purification of nucleic acid according to the item 1, wherein the surfactant contained in said pretreatment solution is a nonionic surfactant.
3. The method for separation and purification of nucleic acid according to the item 2, wherein the nonionic surfactant is a surfactant of a polyoxyethylene type.
4. The method for separation and purification of nucleic acid according to the item 3, wherein the surfactant of a polyoxyethylene type is a surfactant of a polyoxyethylene sorbitan type.
5. The method for separation and purification of nucleic acid according to any one of the items 1 to 4, wherein the sample solution containing nucleic acid in the step (1) is a sample solution prepared by adding a tissue lysis solution to animal tissue to subject to a digesting treatment of said animal tissue.
6. The method for separation and purification of nucleic acid according to any one of the items 1 to 4, wherein the sample solution containing nucleic acid in the aforementioned step (1) is a sample solution containing an anticoagulant and at least one of blood and white blood cell.
7. The method for separation and purification of nucleic acid according to the item 5, wherein the tissue lysis solution is a solution containing at least one of compounds selected from the group consisting of surfactant, buffer, stabilizer for nucleic acid, alkali metal halide, chaotropic agent, protease and antifoaming agent.
8. The method for separation and purification of nucleic acid according to any one of the items 1 to 7, wherein the solid phase comprising the organic macromolecule having a polysaccharide structure is a solid phase comprising acetylcellulose.
9. The method for separation and purification of nucleic acid according to the " item 8, wherein the solid phase comprising acetylcellulose is a solid phase comprising an organic macromolecule prepared by subjecting acetylcellulose or a mixture of acetylcelluloses having different acetyl values to a saponification treatment.
10. The method for separation and purification of nucleic acid according to the item 9, wherein the saponification rate of the mixture of acetylcelluloses having different acetyl values is 5% or more.
11. The method for separation and purification of nucleic acid according to the item 1, wherein the solid phase comprising an organic macromolecule having a polysaccharide structure is a solid phase comprising a regenerated cellulose.
12. The method for separation and purification of nucleic acid according to any one of the items 1 to 11, wherein the solid phase is a porous membrane.
13. The method for separation and purification of nucleic acid according to the item 12, wherein the porous membrane is a porous membrane in which the front and back sides are asymmetric.
14. The method for separation and purification of nucleic acid according to the item 12, wherein the porous membrane is a porous membrane having an average pore size of 0.1 to 10.0 µm.
15. The method, for separation and purification of nucleic acid according to any one of the items 12 to 14, wherein the porous membrane is a porous membrane having a thickness of 10 to 500 µm.
16. The method for separation and purification of nucleic acid according to the items 1 to 15, wherein the water-soluble organic solvent includes at least one of methanol, ethanol, propanol and butanol.
17. The method for separation and purification of nucleic acid according to any one of the items 5 to 16, which comprises removing non-dissolved residual tissue components from the sample solution prepared in the step of adding a tissue lysis solution to animal tissue to subject the animal tissue to a digesting treatment.
18. The method for separation and purification of nucleic acid according to any
   of the items 5 to 17, which comprises adding a solution of RNase to the sample solution prepared in the step of adding a tissue lysis solution to animal tissue to subject animal tissue to a digesting treatment.
19. The method for separation and purification of nucleic acid according to any one of the items 1 to 18, wherein adsorption and desorption of nucleic acid are carried out using a unit for separation and purification of nucleic acid where a solid phase is housed in a container having at least two openings.
20. The method for separation and purification of nucleic acid according to any one of the items 1 to 19, wherein adsorption and desorption of nucleic acid are carried out using a unit for separation and purification of nucleic acid which contains (a) a solid phase, (b) a container having at least two openings, which houses the solid phase and (c) an apparatus for generation of pressure difference being connected to one of the openings of the aforementioned container.
21. The method for separation and purification of nucleic acid according to the item 20, wherein the apparatus for generation of pressure difference is an apparatus for pressurization.
22. The method for separation and purification of nucleic acid according to the item 20, wherein the apparatus for generation of pressure difference is an apparatus for depressurization.
23. The method for separation and purification of nucleic acid according to any one of the items 20 to 22, wherein the apparatus for generation of pressure difference is attached to one of the openings of the container in a freely detachable manner.
24. The method for separation and purification of nucleic acid according to the item 20 or 21, which comprises the following steps.
   (a) adding a pretreatment solution to a sample solution containing nucleic acid to prepare a solution containing nucleic acid for adsorbing to a solid phase;
   (b) injecting a solution containing nucleic acid for adsorbing to solid phase into one of the openings of the unit for separation and purification of nucleic acid;
   (c) making the inside of the container into a pressurized state using an apparatus for generation of pressure difference attached to one of the aforementioned openings of the unit for separation and purification of nucleic acid, and discharging a solution containing nucleic acid for adsorbing to solid phase injected thereinto from another opening, so that the solution is contacted to the solid phase and nucleic acid is adsorbed to the solid phase;
   (d) injection a washing solution into the one of the openings of the unit for separation and purification of nucleic acid;
   (e) making the inside of the container into a pressurized state using an apparatus for generation of pressure difference attached to one of the aforementioned openings of the unit for separation and purification of nucleic acid, and discharging the washing solution injected thereinto from the another opening, so that the washing solution is contacted to the solid phase and the solid phase is washed;
   (f) injecting an elution solution which is able to desrob the nucleic acid adsorbed with the solid phase into the another opening of the unit for separation and purification of nucleic acid; and
   (g) making the inside of the container into a pressurized state using an apparatus for generation of pressure difference attached to the one of the openings of the unit for separation and purification of nucleic acid, and discharging the elution solution injected thereinto from the another opening, so that nucleic acid adsorbed with the solid phase is desorbed and discharged to the outside of the container.
25. The method for separation and purification of nucleic acid according to the item 24, which, before the aforementioned step (f), comprises contacting a DNase solution to the solid phase and then washing the solid phase with a washing solution.
26. The method for separation and purification of nucleic acid according to any one of the items 1 to 25, wherein the washing solution is a solution containing 20 to 100% by weight of methanol, ethanol, isopropanol or n-propanol.
27. The method for separation and purification of nucleic acid according to any one of the items 1 to 26, wherein the elution solution is a solution having a salt concentration of 0.5 mol/L or less.
28. A reagent kit for conducting the method mentioned in any one of the items 1 to 27, comprising: (i) an unit for separation and purification of nucleic acid, (ii) protease, (iii) a chaotropic salt, surfactant and BisTris, (iv) a washing solution, (v) a reagent for a elution solution, and (vi) a water-soluble organic solvent.

In accordance with the method of the present invention, it is now possible to separate nucleic acid of high purity in a high efficiency from a sample solution containing nucleic acid prepared, particularly from animal cells.

### Brief Description of the Drawings

Fig. 1 is a drawing which was prepared by subjecting nucleic acid separated and purified according to the method of the present invention and a marker for molecular weight to electrophoresis.
Fig. 2 is a drawing which was prepared by subjecting nucleic acid separated and purified according to the method of the present invention, nucleic acid separated and purified as a Comparative Example and a marker for molecular weight to pulse-field electrophoresis.
Fig. 3 is a drawing which was prepared by subjecting nucleic acid separated and purified according to the method of the present invention and a marker for molecular weight to electrophoresis.

### Mode for Carrying out the Invention

A method for separation and purification of nucleic acid according to the present invention will be specifically illustrated. In the present invention, a sample solution containing nucleic acid, preferably prepared from animal, tissue, is contacted to a solid phase whereupon nucleic acid in the sample solution is adsorbed with the solid phase and then nucleic acid adsorbed with the solid phase is desorbed from the solid phase using an appropriate solution which will be mentioned below. Preferably, the sample solution containing nucleic acid prepared from animal tissue is a solution where the animal tissue is treated with a tissue lysis solution (digesting solution for tissues) containing at least one compound selected from surfactant, buffer, stabilizer for nucleic acid, alkali metal halide, protease and antifoaming agent and then a pretreatment solution containing at least one compound selected from antifoaming agent, stabilizer for nucleic acid, chaotropic salt, surfactant and buffer being BisTris (bis(2-hydroxyethyl)iminotris(hydroxymethyl)methane) is added thereto and that is prepared by further addition of a water-soluble organic solvent thereto.

Although there is no limitation for animal tissues which are able to be used in the present invention, animal tissue such as kidney, liver, spleen, heart, lung and brain and a part thereof or a part of body of animal such as tail and ear is used as an object. Components of blood and lymph are also able to be used as an object. Cells and liquid and solid components separated from animal tissue are also able to be used as an object. Incubated cells, cells which are able to conduct an autonomous growth and particles which are able to grow in cells are also able to be used as an object. Product which is collected by rubbing of animal tissue is also able to be used as an object.

The sample may be frozen or may be used as it is. A product which is preserved by freezing may be used as well and a product which is preserved in a preserving solution, preserved in thin flakes and embedded in resin or the like may also be used.

Further, the sample.of the present invention may contain an anticoagulant. With regard to the anticoagulant, general examples thereof are EDTA, heparin, sodium citrate, sodium fluoride and ACD (acid citrate dextrose solution) and each of them may be used solely or a combination of two or more thereof may be used jointly. The amount may be used within a common using amount. Nucleic acid is able to be efficiently separated and purified regardless of those anticoagulants and regardless of the type of the anticoagulant contained in the sample.

Since animal tissue is usually solid, it is necessary that it is dissolved by a treating solution or is physically destructed. In the present invention, such an animal tissue is firstly dissolved by treating with a tissue lysis solution. At that time, it is preferred that the animal tissue is previously made into small pieces by cutting or freeze-pulverizing. For example, the treatment is conducted in such a manner that a tissue lysis solution is added to the animal tissue as above and dissolving is conducted by warming the temperature of the solution at 30 to 75°C, preferably at 40 to 65°C or, more preferably, at 50 to 60°C. Thus, for example, it is conducted by such a manner that the solution is allowed to stand with stirring using a shaker or the like for several hours to one night or several days by keeping at 55°C. It is also possible that it is allowed to stand together with occasional stirring. Depending upon the dissolving state of the tissue, it is also possible to change the composition concentration of the dissolving solution for tissues or to change treating temperature, stirring numbers and treating time. It is further possible to dip in a solution for dissolving the tissues followed by being allowed to stand. As to another method, it is also possible to utilize a step of making the animal tissue into fragments using physical means such as mill or homogenizer.

It is also possible to preserve the sample with a dissolving solution for tissues.

One standard is that 1 to 2,000 µl of the solution for dissolving the tissues is able to be used for 0.1 to 200 mg of the tissue although that is able to be increased or decreased depending upon the state of the tissue. In the case of liquid separated from the tissue, 1 to 2,000 µl may be used for 1 µl to 2 ml although that is able to be increased or decreased depending upon the state of the liquid. In the case of cells or particles, 1 to 2,000 µl may be used for 10 to 1 × 10⁸ cells/particles although that is able to be increased or decreased depending upon the state of cells or particles.

Although its pH may be within a range of 4 to 12, it is preferably 6 to 11 and, more preferably, 7.5 to 9.5.

As mentioned above, a solution prepared by treating the animal tissue with a dissolving solution for tissues is used. When undissolved residual tissue components remain in that solution, it is preferred that they are removed by centrifugal separation, filtration using a filter, etc.

When the aimed nucleic acid to be recovered is DNA, it is also possible that a solution of RNase is added to the aforementioned solution prepared by treating the animal tissue with a dissolving solution for tissues whereby RNA is previously decomposed. When the aimed nucleic acid is RNA, it is also possible that a solution of DNase is added to the aforementioned solution prepared by treating the animal tissue with a dissolving solution for tissues whereby DNA is previously decomposed.

The enzyme solution may contain a buffer, may contain univalent or divalent alkali metal salt or may contain a polyhydric alcohol such as glycerol.

For example, 0.1 to 100 µl of RNase of 10 to 100 mg/ml may be added to 200 µl of a dissolving solution for tissues although that may be increased or decreased depending upon the state of the sample. Preferably, 100 mg/ml of RNase may be added in an amount of 1 to 50 µl to 200 µl of the dissolving solution for tissues.

Such an enzyme may be natural products or recombinants and plural enzymes may be used by mixing.

Nucleic acid of the animal tissue solution which is treated with the above solution for dissolving the tissues is treated with a pretreatment solution for making it soluble. As a result, cell membrane and nucleus membrane are further dissolved and nucleic acid is dispersed in a sample solution.

As a standard, the pretreatment solution may be used in an amount of 1 to 2,000 µl for 0.1 to 200 mg of the dissolved tissue although that may be increased or decreased depending upon the state of the tissue. In the case of solution separated from the tissue, 1 to 2,000 µl may be used for 1 µl to 2 ml although that is able to be increased or decreased depending upon the state of the solution. In the case of cells or particles, 1 to 2,000 µl may be used for 10 to 1 × 10⁸ although that is able to be increased or decreased depending upon the state of cells or particles.

Although its pH may be within a range of 3 to 12, it is preferably 4 to 8 and, more preferably, 5 to 7.

When the reagent is mixed with a treating solution for tissues, stirring may be conducted for 1 second to 30 minutes at 30 to 3,000 rpm using a stirring device. It is also possible to conduct a tumbling mixing for 1 to 30 times. It is also possible to conduct a pipetting operation for 1 to 50 times. It is also possible to conduct a tapping operation for 1 to 50 times. With regard to a method for mixing, two or more thereof may be conducted jointly. It is preferred to mix well.

After the reagent and the tissue treating solution are mixed, they may be heated. For example, they may be heated at 30 to 95°C for 1 to 60 minute(s). Preferably, they may be heated at 50 to 75°C for 1 to 15 minute(s).

In the present invention, "nucleic acid" may be single-stranded or double-stranded and may be any of RNA and RNA. There is no limitation for molecular weight thereof as well.

The sample means any sample containing nucleic acid. Type of nucleic acid in the sample may be one or plural of two or more. There is also no particular limitation for the length of each nucleic acid and, for example, nucleic acid of any length from several bp to several Mbp may be used. In view of handling, length of nucleic acid is usually about several bp to several hundred kbp.

Specific examples of the surfactant used in the present invention are nonionic surfactant, anionic surfactant, cationic surfactant and amphoteric surfactant which may be used either solely or jointly. Concentration thereof which may be used is able to be selected from the range of 0.001% to 99.99% in the dissolving solution.

In the present invention, anionic surfactant and nonionic surfactant may be preferably used.

Examples of the anionic surfactant are surfactant of a sulfate type, surfactant of a sulfonic acid type, surfactant of a carboxylic acid type and surfactant of a phosphoric acid type. Alkyl sulfate salt is preferred. Sodium dodecylsulfate is particularly preferred. Preferably, it is contained in a dissolving solution for tissues and is made to act for dissolving of tissues. Anionic surfactant may be added to a dissolving solution in an amount of 0.01% to 50%. Preferably, it is selected within a range of 0.05% to 5%.

Examples of the nonionic surfactant are surfactant of a polyoxyethylene alkyl phenyl ether type, surfactant of a polyoxyethylene alkyl ether type and fatty acid alkanol amide and preferred one is a surfactant of a polyoxyethylene alkyl ether type. Among the surfactant of a polyoxyethylene alkyl ether type, more preferred ones are POE decyl ether, POE lauryl ether, POE tridecyl ether, POE alkylene decyl ether, POE sorbitan monolaurate, POE sorbitan monooleate, POE sorbitan monostearate, polyoxyethylene sorbitol tetraoleate, POE alkylamine and POE acetylene glycol. Nonionic surfactant is able to be added to a dissolving solution in an amount of 0.01% to 50%. Preferably, it is able to be selected within a range of 0.05% to 5%.

Those surfactants may be used either solely or jointly by combining plural ones. Concentration of the surfactant in the aforementioned dissolving solution for tissues and in the pretreatment solution is preferred to be 0.1 to 20% by mass. More preferably, selection is able to be done within a range that anionic surfactant is contained in an amount of 0.05% to 5% in a dissolving solution and that nonionic surfactant is contained in an amount of 0.05% to 5% in a dissolving solution.

With regard to the specific examples of a buffer used in the present invention, commonly used pH buffers may be listed. Preferably, pH buffers for biochemistry may be listed. Examples of the buffers as such are a buffer comprising citrate, phosphate or acetate, Tris-HCl, TE (Tris-HCl/EDTA), TBE (Tris-Borate/EDTA), TAE (Tri-Acetate/EDTA) and Good's buffer. Examples of Good's buffer are MES (2-morpholinoethanesulfonic acid), Bis-Tris (bis(2-hydroxyethyl)iminotris(hydroxymethyl)methane, HEPES (2-[4-(2-hydroxyethyl)-1-piperazinyl]- ethanesulfonic acid), PIPES (piperaxine-1,4-bis(2-ethane- sulfonic acid), ACES (N-(2-acetamino)-2-aminoethanesulfonic acid), CAPS (N-cyclohexyl-3-aminopropanesulfonic acid) and TES (N-tns(hydroxymethyl)methyl-2-aminoethanesulfonic acid). The pretreatment solution contains BisTris as a buffer.

It is preferred that concentration of the buffer in the aforementioned dissolving solution for tissues and in the pretreatment solution is 1 to 300 mM. More preferably, the concentration is able to be selected within a range of 20 mmol/l to 150 mmol/l.

As the nucleic acid stabilizing agent, one having a reaction to inactivate a nuclease activity can be exemplified. Depending on a test sample, there are cases where nuclease, which degrades nucleic acid, is comprised thereto so that when nucleic acid is homogenized, nuclease reacts with nucleic acid, so as to result in a remarkable reduction of a yield amount. For the purpose of avoiding this, a stabilizing agent having a function to inactivate nuclease can be coexisted in a nucleic acid-solubilizing solution. As a result, improvements in a recovering yield and a recovering efficiency of nucleic acid lead to the minimization and acceleration of a test sample.

As the nucleic acid stabilizing agent having functions to inactivate the nuclease activity, a compound used routinely as a reducing agent can be used. Examples of reducing agents include hydrogenated compounds such as a hydrogen atom, hydrogen iodide, hydrogen sulfide, aluminum lithium hydride, and sodium borohydride; a highly electropositive metal such as alkaline metal, magnesium, calcium, aluminum, and zinc, or their amalgam; organic oxides such as aldhyde-based, sugar-based, formic acid, and oxalic acid; and mercapto compounds. Among these, the mercapto compounds are preferable. Examples of mercapto compounds include N-acetyl cysteine, mercapto ethanol, and alkyl mercaptane or the like. The mercapto compounds can be used alone or in combinations of two or more. The concentration of the nucleic acid stabilizing agent in the nucleic acid-solubilizing reagent is preferably from 0.1 to 20% by weight, and more preferably from 0.3 to 15% by weight.

As a stabilizing agent for nucleic acid having an action of inactivating the activity of nuclease, a chelating agent may be used. With regard to a chelating agent, EDTA, NTA, EGTA, etc. may be listed. The chelating agent may be used either solely or jointly by combining plural ones. For example, EDTA may be used within a range of action concentration of 1 to 300 mmol/l. More preferably, it may be selected from the concentration range of 10 mmol/l to 150 mmol/1. In addition, it may be preferably contained in a dissolving solution for tissues and is made to act for inactivation of endogenous nuclear activity.

Specific examples of halide of alkaline metal used in the present invention are halides, preferably chlorides, of sodium, potassium and lithium.

The halide of alkaline metal may be used in a concentration of 1 to 200 mmol/l in the aforementioned dissolving solution for tissues and in the pretreatment solution.

Specific examples of the protease used in the present invention are serine protease, cysteine protease and metal protease and at least one protease may be preferably used. With regard to the protease, a mixture of plural proteases may be preferably used as well.

There is no particular limitation for serine protease and, for example, protease K may be used preferably. There is no particular limitation for cysteine protease and, for example, papain and cathepsin may be preferably used. There is no particular limitation for metal protease and, for example, carboxypeptitase may be used preferably. Concentration of the protease in the aforementioned solution for dissolving the tissue per 1 ml of total volume upon addition is preferably 0.001 IU to 10 IU or, more preferably, 0.01 IU to 1 IU. Alternatively, it may be used 0.05 to 20 mg/ml as an acting concentration.

Such an enzyme may be a natural substance or a recombinant and may be used by mixing.

In order to stably maintain the action of the protease, a buffer may be added to a dissolving solution for tissues. For example, 1 to 200 ml/l of Tris HCl may be contained therein.

With regard to the protease, a protease having no nuclease may be preferably used. Protease containing a stabilizer may be preferably used as well. With regard to a stabilizer, metal ion may be used preferably. To be more specific, magnesium ion and calcium ion are preferred and it is possible to add, for example, in a form of magnesium chloride or calcium acetate. When a stabilizer for protease is contained therein, it is possible to make the amount of protease necessary for recovery of nucleic acid very small and to reduce the cost necessary for recovery of nucleic acid. It is possible that a buffer is made contained in a protease solution or a polyhydric alcohol is added thereto. For example, as a buffer, Tris HCl may be contained in an amount of 0.1 to 200 ml/l or glycerol may be contained in an amount of 1 to 70%. Such a substance may be use either solely or jointly by combination.

As the defoaming agent, a silicon-based defoaming agent (e.g., silicon oil, dimethyl polysiloxane, silicon emersion, denatured polysiloxane, silicon compound, etc.), alcohol-based defoaming agent (e.g., acetylene glycol, heptanol, ethyl exanol, superhigh grade alcohol, polyoxy alkylene glycol, etc.), ether-based defoaming agent (e.g., heptyl cellosolve, nonyl cellosolve-3-heptylcorbitol, etc.), fatty oil-based defoaming agent (e.g., animal and plant fat, etc.), fatty acid-based defoaming agent (e.g., stearic acid, oleic acid, palmitic acid, etc.), metallic soap-based defoaming agent (e.g., aluminum stearate, calcium stearate, etc.), fatty acid ester-based defoaming agent (e.g., a natural wax, tributyl phosphate, etc.), phosphate ester-based defoaming agent (e.g., sodium octyl phosphate, etc.), amine-based defoaming agent (e.g., diamyl amine, etc.), amide-based defoaming agent (e.g., amide stearate, etc.), and other defoaming agents (e.g., ferric sulfate, bauxite, etc.) can be exemplified. These defoaming agent can be used alone or in combinations of two or more. Two compounds combined from silicon-based and alcohol-based defoaming agents are especially preferred. As alcohol-based defoaming agents, acetylene glycol-based surfactant is also preferable. The concentration of a defoaming agent in the reagent is preferably in a range of 0 to 10% by weight, more preferably 0.05 to 2% by weight.

As specific examples of the chaotropic agent used in the present invention, guanidine salt, sodium isocyanate, sodium iodide, potassium iodide, etc. may be used. Among them, guanidine salt is preferred. Examples of the guanidine salt are guanidine hydrochloride, guanidine isothiocyanate and guanidine thiocyanate and, among them, guanidine hydrochloride is preferred. Such a salt may be used either solely or jointly by combining two or more thereof. Concentration of the chaotropic salt in the aforementioned solution for dissolving of tissues, pretreatment solution or purified nucleic acid solution is preferably not less than 0.5 mol/1, more preferably 0.5 mol/l to 4 mol/l and, still more preferably, 1 mol/l to 3 mol/l.

Urea may also be used as a chaotropic agent instead of a chaotropic salt. Urea may be selected within a range of from 0 to 10 mol/1.

After that, a water-soluble organic solvent is added to a sample solution in which nucleic acid is dispersed and contacted to the solid phase. As a result of this operation, nucleic acid in the sample solution is adsorbed with the solid phase. In order to adsorb the nucleic acid which is solubilized by the aforementioned operation in this specification with the solid phase, it is necessary that the mixed solution of solubilized nucleic acid is mixed with the water-soluble organic solvent and also that salt is present in the resulting mixed solution of nucleic acid.

Thus, when hydrated structure of water molecule existing around the nucleic acid is destructed, nucleic acid is solubilized in an unstable state. It is believed that, when nucleic acid in such a state is contacted to a solid phase comprising an organic macromolecule having hydroxyl groups on the surface, an interaction takes place between the polar group on the surface of nucleic acid and the polar group on the surface of the solid whereupon nucleic acid is adsorbed on the surface of the solid phase. In the method of the present invention, nucleic acid is made into an unstable state by mixing a water-soluble organic solvent with the mixed solution of solubilized nucleic acid and by the presence of a salt in the resulting mixed solution of nucleic acid.

Examples of such a water-soluble organic solvent are alcohol, acetone, acetonitrile and dimethylformamide. Among them, alcohol is preferred. With regard to the alcohol, any of primary alcohol, secondary alcohol and tertiary alcohol may be used. Among them, methanol, ethanol, propanol and isomers thereof and butanol and derivatives thereof may be listed and ethanol is more preferred.

The final concentration of such a water-soluble organic solvent in the sample solution containing nucleic acid is preferred to be 5 to 90% by mass. More preferably, it is 20% by weigh to 60% by weight. With regard to the adding concentration of ethanol, it is particularly preferred to be as high as possible within such an extent that no aggregate is formed.

As a standard, the water-soluble organic solvent may be used in an amount of 1 to 2,000 µl for 0.1 mg to 200 mg of the dissolved tissue although that may be increased or decreased depending upon the state of the tissue. In the case of liquid separated from the tissue, 1 to 2,000 µl may be used for 1 µl to 2 ml although that is able to be increased or decreased depending upon the state of the liquid. In the case of cells or particles, 1 to 2,000 µl may be used for 10 to 1 × 10⁸ cells/particles although that is able to be increased or decreased depending upon the state of cells or particles.

The water-soluble solvent may be added after mixing with the aforementioned treated solution or may be added solely. It is also possible to mix followed by adding and then to further add solely.

When the solvent is mixed with a treating solution for tissues, stirring may be conducted for 1 second to 30 minutes at 30 to 3,000 rpm using a stirring device. It is also possible to conduct a tumbling mixing for 1 to 30 times. It is also possible to conduct a pipetting operation for 1 to 50 times. It is also possible to conduct a tapping operation for 1 to 50 times. With regard to a method for mixing, two or more may be conducted jointly. It is preferred to mix well.

With regard to a salt existing in the resulting mixed solution of nucleic acid, preferred ones are various kinds of chaotropic substances (guanidium salt, sodium iodide, sodium perchlorate), sodium chloride, potassium chloride, ammonium chloride, sodium bromide, potassium bromide, calcium bromide, ammonium bromide, etc.

With regard to the aforementioned sample solution used, its pH is preferably 3 to 10, more preferably 4 to 9 and, still more preferably, 5 to 8.

Although its temperature may be 5°C to 50°C, it may be selected from the range of preferably 10°C to 40°C or, more preferably, 15°C to 35°C.

With regard to the resulting sample solution containing nucleic acid, it is preferred to be within such ranges that its surface tension is 00.5 J/m² or less, it viscosity is 1 to 10,000 mPa and its specific gravity is 0.8 to 1.2. When the solution is made within the aforementioned ranges, it is easy to remove the remaining solution after adsorption of nucleic acid by passing a sample solution containing nucleic acid through the aforementioned solid phase in the next step.

With regard to the solid phase, it is preferred to be a solid phase where nucleic acid is adsorbed by an interaction in which ionic bond is not substantially participated. That means the fact that no "ionization" takes place in the condition for the use of the solid phase and it is presumed that, when polarity of the environment is changed, nucleic acid and solid phase pull against each other. As a result, it is now possible that nucleic acid is isolated and purified in an excellent separating property and in a high washing efficiency. That is presumably because of the solid phase having hydrophilic group and, when polarity of the environment is changed, hydrophilic groups of both nucleic acid and solid phase pull against each other.

The hydrophilic group means a polar group (atoms) capable of exerting an interaction with water, and includes all groups (atoms) participating in adsorption of nucleic acid. As the hydrophilic group, those which exhibit about a middle level of interaction with water (see, "group having not so strong hydrophilicity" in the item of "hydrophilic group" described in Kagaku Dai-jiten, published by Kyoritsu Shuppan) are preferred, and examples thereof include a hydroxyl group, a carboxyl group, a cyano group and a hydroxyethyl group, with a hydroxyl group being preferred.

Here, the term "solid phase having a hydrophilic group" means a solid phase wherein the material constituting the solid phase itself has the hydrophilic group, or a solid phase obtained by treating or coating a solid phase-constituting material in order to introduce the hydrophilic group into the solid phase. The solid phase-constituting material may be an organic or inorganic material. For example, there may be used a solid phase wherein the solid phase-constituting material itself is an organic material having a hydrophilic group, a solid phase which is obtained by treating a solid phase made of a hydrophilic group-free organic material so as to introduce the hydrophilic group thereinto, a solid phase obtained by coating a solid phase made of a hydrophilic group-free organic material with a material having a hydrophilic group to thereby introduce the hydrophilic group, a solid phase wherein the solid phase-constituting material itself is an inorganic material having a hydrophilic group, a solid phase which is obtained by treating a solid phase made of a hydrophilic group-free inorganic material so as to introduce the hydrophilic group thereinto, and a solid phase obtained by coating a solid phase made of a hydrophilic group-free inorganic material with a material having a hydrophilic group to thereby introduce the hydrophilic group. In view of processing ease, it is preferable to use an organic material such as an organic polymer as the material for constituting the solid phase.

With regard to the solid phase of an organic material having hydrophilic group which is able to be used in the present invention, its examples includes solid phase formed by polyhydroxyethylacrylic acid, polyhydroxyethylmethacrylic acid, polyvinyl alcohol, polyvinylpyrrolidone, polyacrylic acid, polymethacrylic acid and polysaccharide such as polyoxyethylene and solid phase of an organic material having a polysaccharide structure is able to be used particularly preferably.

As the organic material having a polysaccharide structure, cellulose, hemicellulose, dextran, amylase, amylopectin, starch, glycogen, pullulan, mannan, glucomannan, lichenan, isolichenan, laminaran, carrageenan, xylan, fructan, alginic acid, hyaluronic acid, chondroitin, chitin and chitosan can preferably be used. However, these are not limitative, and any organic material having a polysaccharide structure or its derivative may be used. Also, an ester derivative of any of these polysaccharides can preferably be used. Further, a saponification product of the ester derivative of any of these polysaccharides can preferably be used.

As the ester of the ester derivative of any of the above-mentioned polysaccharides, one or more members selected from among carboxylates, nitrates, sulfates, sulfonates, phosphates, phosphonates and pyrophosphates are preferably selected. Also, saponification products of the carboxylates, nitrates, sulfates, sulfonates, phosphates, phosphonates and pyrophosphates can more preferably be used.

As the carboxylates of any of the above-mentioned polysaccharides, one or more members selected from among alkylcarbonyl esters, alkenylcarbonyl esters, aromatic carbonyl esters and aralkylcarbonyl esters are preferably selected. Also, saponification products of the alkylcarbonyl esters, alkenylcarbonyl esters, aromatic carbonyl esters and aralkylcarbonyl esters of any of the above-mentioned polysaccharides can more preferably be used.

As the ester group of the alkylcarbonyl esters of any of the above-mentioned polysaccharides, one or more members selected from among an acetyl group, a propionyl group, a butyroyl group, a valeryl group, a heptanoyl group, an octanoyl group, a decanoyl group, a dodecanoyl group, a tridecanoyl group, a hexadecanoyl group and an octadecanoyl group are preferably selected. Also, saponification products of any of the above-mentioned polysaccharides having one or more ester groups selected from among an acetyl group, a propionyl group, a butyloyl group, a valeryl group, a heptanoyl group, an octanoyl group, a decanoyl group, a dodecanoyl group, a tridecanoyl group, a hexadecanoyl group and an octadecanoyl group can more preferably be used.

As the ester group of the alkenylcarbonyl esters of any of the above-mentioned polysaccharides, one or more of an acryl group and a methacryl group are preferably selected. Also, saponification products of any of the above-mentioned polysaccharides having ester groups of one or more of an acyl group and a methacryl group can more preferably be used.

As the ester group of the aromatic carbonyl esters of any of the above-mentioned polysaccharides, one or more of a benzoyl group and a naphthaloyl group are preferably selected. Also, saponification products of any of the above-mentioned polysaccharides having ester groups of one or more of a benzoyl group and a naphthaloyl group can more preferably be used.

As the nitrates of any of the polysaccharides, nitrocellulose, nitrohemicellulose, nitrodextran, nitroagarose, nitrodextrin, nitroamylase, nitroamylopectin, nitroglycogen, nitropullulan, nitromannan, nitroglucomannan, nitrolichenan, nitroisolichenan, nitrolaminaran, nitrocarrageenan, nitroxylan, nitrofructan, nitroalginic acid, nitrohyaluronic acid, nitrochondroitin, nitrochitin and nitrochitosan can preferably be used.

Also, saponification products of nitrocellulose, nitrohemicellulose, nitrodextran, nitroagarose, nitrodextrin, nitroamylase, nitroamylopectin, nitroglycogen, nitropullulan, nitromannan, nitroglucomannan, nitrolichenan, nitroisolichenan, nitrolaminaran, nitrocarrageenan, nitroxylan, nitrofructan, nitroalginic acid, nitrohyaluronic acid, nitrochondroitin, nitrochitin and nitrochitosan can more preferably be used.

As the sulfates of any of the polysaccharides, cellulose sulfate, hemicellulose sulfate, dextran sulfate, agarose sulfate, dextrin sulfate, amylase sulfate, amylopectin sulfate, glycogen sulfate, pullulan sulfate, mannan sulfate, glucomannan sulfate, lichenan sulfate, isolichenan sulfate, laminaran sulfate, carrageenan sulfate, xylan sulfate, fructan sulfate, alginic acid sulfate, hyaluronic acid sulfate, chondroitin sulfate, chitin sulfate and chitosan sulfate can preferably be used.

Also, saponification products of cellulose sulfate, hemicellulose sulfate, dextran sulfate, agarose sulfate, dextrin sulfate, amylase sulfate, amylopectin sulfate, glycogen sulfate, pullulan sulfate, mannan sulfate, glucomannan sulfate, lichenan sulfate, isolichenan sulfate, laminaran sulfate, carrageenan sulfate, xylan sulfate, fructan sulfate, alginic acid sulfate, hyaluronic acid sulfate, chondroitin sulfate, chitin sulfate and chitosan sulfate can more preferably be used.

As the sulfonates of any of the aforementioned polysaccharides, one or more members selected from among alkyl sulfonates, alkenyl sulfonates, aromatic sulfonates and aralkyl sulfonates are preferably selected. Also, saponification products of alkyl sulfonates, alkenyl sulfonates, aromatic sulfonates and aralkyl sulfonates of any of the above-mentioned polysaccharides can more preferably be used.

As the phosphates of any of the aforementioned polysaccharides, cellulose phosphate, hemicellulose phosphate, dextran phosphate, agarose phosphate, dextrin phosphate, amylase phosphate, amylopectin phosphate, glycogen phosphate, pullulan phosphate, mannan phosphate, glucomannan phosphate, lichenan phosphate, isolichenan phosphate, laminaran phosphate, carrageenan phosphate, xylan phosphate, fructan phosphate, alginic acid phosphate, hyaluronic acid phosphate, chondroitin phosphate, chitin phosphate and chitosan phosphate can preferably be used.

Also, saponification products of cellulose phosphate, hemicellulose phosphate, dextran phosphate, agarose phosphate, dextrin phosphate, amylase phosphate, amylopectin phosphate, glycogen phosphate, pullulan phosphate, mannan phosphate, glucomannan phosphate, lichenan phosphate, isolichenan phosphate, laminaran phosphate, carrageenan phosphate, xylan phosphate, fructan phosphate, alginic acid phosphate, hyaluronic acid phosphate, chondroitin phosphate, chitin phosphate and chitosan phosphate can more preferably be used.

As the phosphonates of any of the aforementioned polysaccharides, cellulose phosphonate, hemicellulose phosonphate, dextran phosphonate, agarose phosphonate, dextrin phosphonate, amylase phosphonate, amylopectin phosonphate, glycogen phosphonate, pullulan phosphonate, mannan phosphonate, glucomannan phosphonate, lichenan phosphonate, isolichenan phosphonate, laminaran phosphonate, carrageenan phosphonate, xylan phosphonate, fructan phosphonate, alginic acid phosphonate, hyaluronic acid phosphonate, chondroitin phosphonate, chitin phosphonate and chitosan phosphonate can preferably be used.

Also, saponification products of cellulose phosphonate, hemicellulose phosphonate, dextran phosphonate, agarose phosphonate, dextrin phosphonate, amylase phosphonate, amylopectin phosphonate, glycogen phosphonate, pullulan phosphonate, mannan phosphonate, glucomannan phosphonate, lichenan phosphonate, isolichenan phosphonate, laminaran phosphonate, carrageenan phosphonate, xylan phosphonate, fructan phosphonate, alginic acid phosphonate, hyaluronic acid phosphonate, chondroitin phosphonate, chitin phosphonate and chitosan phosphonate can more preferably be used.

As the pyrophosphates of any of the aforementioned polysaccharides, cellulose pyrophosphate, hemicellulose pyrophosphate, dextran pyrophosphate, agarose pyrophosphate, dextrin pyrophosphate, amylase pyrophosphate, amylopectin pyrophosphate, glycogen pyrophosphate, pullulan pyrophosphate, mannan pyrophosphate, glucomannan pyrophosphate, lichenan pyrophosphate, isolichenan pyrophosphate, laminaran pyrophosphate, carrageenan pyrophosphate, xylan pyrophosphate, fructan pyrophosphate, alginic acid pyrophosphate, hyaluronic acid pyrophosphate, chondroitin pyrophosphate, chitin pyrophosphate and chitosan pyrophosphate can preferably be used.

Also, saponification products of cellulose pyrpphosphate, hemicellulose pyrophosphate, dextran pyrophosphate, agarose pyrophosphate, dextrin pyrophosphate, amylase pyrophosphate, amylopectin pyrophosphate, glycogen pyrophosphate, pullulan pyrophosphate, mannan pyrophosphate, glucomannan pyrophosphate, lichenan pyrophosphate, isolichenan pyrophosphate, laminaran pyrophosphate, carrageenan pyrophosphate, xylan pyrophosphate, fructan pyrophosphate, alginic acid pyrophosphate, hyaluronic acid pyrophosphate, chondroitin pyrophosphate, chitin pyrophosphate and chitosan pyrophosphate can more preferably be used.

As the ether derivatives of any of the aforementioned polysaccharides, methyl cellulose, ethyl cellulose, carboxymethyl cellulose, carboxyethyl cellulose, carboxyethyl-carbamoylethyl cellulose, hydroxymethyl cellulose, hydroxyethyl cellulose, hydroxypropyl cellulose, hydroxypropylmethyl cellulose, hydroxyethylmethyl cellulose, cyanoethyl cellulose and carbamoyethyl cellulose can be used, though the ether derivatives not being limited thereto. It is preferable to use hydroxymethyl cellulose or hydroxyethyl cellulose.

Those wherein hydroxyl groups of any of the polysaccharides are halogenated with any substitution degree can also be preferably used.

With regard to a solid phase comprising an organic macromolecule having a polysaccharide structure, acetylcellulose may be listed and a solid phase comprising an organic macromolecule comprising a mixture of acetylcelluloses having different acetyl values may be used as well. With regard to a mixture of acetylcelluloses having different acetyl values, preferably used ones are a mixture of triacetylcellulose and diacetylcellulose, a mixture of triacetylcellulose and monoacetylcellulose, a mixture of triacetylcellulose, diacetylcellulose and monoacetylcellulose and a mixture of diacetylcellulose and monoacetylcellulose. A mixture of triacetylcellulose and diacetylcellulose is particularly preferably used. Mixing ratio (ratio by mass) of triacetylcellulose to diacetylcellulose is preferably from 99:1 to 1:99 and, more preferably, it is from 90:10 to 50:50.

With regard to the particularly preferred solid phase comprising acetylcellulose, a surficially saponified product of acetylcellulose mentioned in Japanese Patent Laid-Open No. 2003/128,691 may be listed. The surficially saponified product of acetylcellulose is a product where a mixture of acetylcelluloses having different acetyl values is subjected to a saponification treatment and preferably used ones are a saponified product of a mixture of triacetylcellulose and diacetylcellulose, a saponified product of a mixture of triacetylcellulose and monoacetylcellulose, a saponified product of a mixture of triacetylcellulose, diacetylcellulose and monoacetylcellulose and a saponified product of a mixture of diacetylcellulose and monoacetylcellulose. It is more preferred to use a saponified product of a mixture of triacetylcellulose and diacetylcellulose. It is preferred that the mixing ratio (ratio by mass) of triacetylcellulose to diacetylcellulose is from 99:1 to 1:99. More preferably, the mixing ratio of triacetylcellulose to diacetylcellulose is from 90:10 to 50:50. In that case, amount (density) of hydroxyl group on the surface of the solid phase is able to be controlled by the degree of the saponification treatment (saponification rate). In order to enhance the separating efficiency of nucleic acid, it is preferred that the amount (density) of hydroxyl group is much more. Saponification rate (surface saponification rate) of the solid phase prepared by the saponification treatment is preferably 5% to 100% and, more preferably, 10% to 100%. In order to make the surface area of the solid phase having hydroxyl group, it is preferred that the solid phase of acetylcellulose is subjected to a saponification treatment. Although the solid phase may be a porous membrane in which the two sides are symmetric, it is preferred to use a porous membrane in which the two sides are asymmetric.

Herein, the saponification treatment means that acetyl cellulose comes in contact with saponification treatment solution (e.g., Sodium hydroxide solution). As a result, the saponification treatment solution contacted ester group of ester derivative of acetyl cellulose is hydrolyzed, and a hydroxyl group is introduced to form regenerated cellulose. Thereby the prepared regenerated cellulose is different in crystalline form from the original cellulose. In order to change the surface saponification degree, saponification treatment is conducted having changed the concentration or treating time of sodium hydroxide. The surface saponification degree is determined by means of NMR, IR or XPS (e.g., detecting a degree of reduction in the peak of carbonyl group).

A method for introducing a hydrophilic group to a solid phase comprising organic material not having a hydrophilic group is to bond a graft polymer chain having a hydrophilic group in inner polymer strand or a side chain to a solid phase.

A method for bonding a graft polymer chain to an organic material of a solid phase include two methods such as a method for chemically bonding a solid phase with graft polymer chain, and a method for polymerizing a compound having a double bond capable of polymerization using a solid phase as a starter to form graft polymer chain.

Firstly, in the method in which the solid phase and graft polymer chain are chemically bonded, a polymer having a functional group capable of reacting with the solid phase in the terminus or side chain of the polymer is used, and they are grafted through a chemical reaction of this functional group with a functional group of the solid phase. The functional group capable of reacting with the solid phase is not particularly limited with the proviso that it can react with a functional group of the solid phase, and its examples include a silane coupling group such as alkoxysilane, isocyanate group, amino group, hydroxyl group, carboxyl group, sulfonate group, phosphate group, epoxy group, allyl group, methacryloyl group, acryloyl group and the like.

Examples of the compound particularly useful as the polymer having a reactive functional group in the terminus or side chain of the polymer include a polymer having trialkoxysilyl group in the polymer terminus, a polymer having amino group in the polymer terminus, a polymer having carboxyl group in the polymer terminus, a polymer having epoxy group in the polymer terminus and a polymer having isocyanate group in the polymer terminus. The polymer to be used in this case is not particularly limited with the proviso that it has a hydrophilic group which is concerned in the adsorption of nucleic acid, and its illustrative examples include polyhydroxyethyl acrylic acid, polyhydroxyethyl methacrylic acid and salts thereof, polyvinyl alcohol, polyvinyl pyrrolidone, polyacrylic acid, polymethacrylic acid and salts thereof, polyoxyethylene and the like.

The method in which a compound having a polymerizable double bond is made into a graft polymer chain by polymerizing it using the solid phase as the starting point is generally called surface graft polymerization. The surface graft polymerization method means a method in which an active species is provided on the base material surface by plasma irradiation, light irradiation, heating or the like method, and a polymerizable compound having double bond arranged in contact with a solid phase is linked to the solid phase by polymerization.

It is necessary that the compound useful for forming a graft polymer chain linked to the base material has both of two characteristics of having a polymerizable double bond and having a hydrophilic group which is concerned in the adsorption of nucleic acid. As such a compound, any one of the polymers, oligomers and monomers having a hydrophilic group can be used with the proviso that it has a double bond in the molecule. Particularly useful compound is a monomer having a hydrophilic group.

As illustrative examples of the particularly useful monomer having a hydrophilic group, the following monomers can be cited. For example, 2-hydroxyethyl acrylate, 2-hydroxyethyl methacrylate, glycerol monomethacrylate and the like hydroxyl group-containing monomers can be used particularly suitably. In addition, acrylic acid, methacrylic acid and the like carboxyl group-containing monomers or alkali metal salts and amine salts thereof can also be used suitably.

As another method for introducing a hydrophilic group into a solid phase of an organic material having no hydrophilic group, a material having a hydrophilic group can be coated. The material to be used in the coating is not particularly limited with the proviso that it has a hydrophilic group which is concerned in the adsorption of nucleic acid, but is preferably a polymer of an organic material from the viewpoint of easy handling. Examples of the polymer include polyhydroxyethyl acrylate, polyhydroxyethyl methacrylate and salts thereof, polyvinyl alcohol, polyvinyl pyrrolidone, polyacrylic acid, polymethacrylic acid and salts thereof, polyoxyethylene, acetyl cellulose, a mixture of acetyl celluloses having different acetyl values and the like, but a polymer having a polysaccharide structure is desirable.

Alternatively, it is possible to coat acetyl cellulose or a mixture of acetyl celluloses having different acetyl values on a solid phase of an organic material having no hydrophilic group and then to subject the coated acetyl cellulose or a mixture of acetyl celluloses having different acetyl values to a saponification treatment. In that case, the saponification ratio is preferably about 5% or more and 100% or less. The saponification ratio is more preferably 10% or more and 100% or less.

As the solid phase of an inorganic material having a hydrophilic group, a solid phase containing a silica compound can be exemplified. As the solid phase containing a silica compound, a glass filter can be exemplified. Also can be exemplified is a porous silica thin membrane described in Japanese Patent No. 3,058,3442. This porous silica thin membrane can be prepared by spreading a developing solution of a cationic amphipathic substance having an ability to form a bimolecular membrane on a base material, preparing multi-layered bimolecular thin membranes of the amphipathic substance by removing the solvent from the liquid membrane on the base material, allowing the multi-layered bimolecular thin membranes to contact with a solution containing a silica compound, and then extracting and removing the aforementioned multi-layered bimolecular thin membranes.

Regarding the method for introducing a hydrophilic group into a solid phase of an inorganic material having no hydrophilic group, there are a method in which the solid phase and a graft polymer chain are chemically bonded and a method in which a graft polymer chain is polymerized using a hydrophilic group-containing monomer having a double bond in the molecule, using the solid phase as the starting point.

When the solid phase and graft polymer chain are attached by chemical bonding, a functional group capable of reacting with a terminal functional group of the graft polymer chain is introduced into an inorganic material, and the graft polymer chain is chemically bonded thereto. Also, when a graft polymer chain is polymerized using a hydrophilic group-containing monomer having a double bond in the molecule and using the solid phase as the starting point, a functional group which becomes the starting point in polymerizing the double bond-containing compound is inserted into the inorganic material.

As the graft polymer having a hydrophilic group and hydrophilic group-containing monomer having a double bond in the molecule, the aforementioned graft polymer having a hydrophilic group and hydrophilic group-containing monomer having a double bond in the molecule, described in the foregoing regarding the method for introducing a hydrophilic group into a porous membrane of an organic material having no hydrophilic group, can be suitably use.

Another method for introducing a hydrophilic group to a solid phase comprising inorganic material not having a hydrophilic group is to coat a material having a hydrophilic group thereon. Materials used in coating are not limited as long as the hydrophilic group participates in the adsorption of nucleic acid, but for easy workability, a polymer of organic material is preferred. Examples of polymer include polyhydroxyethylacrylate, polyhydroxyethylmethacrylate and their salts, polyvinyl alcohol, polyvinylpyrrolidone, polyacrylate, polymethacrylate and their salts, polyoxyethylene, acetyl cellulose, and a mixture of acetyl celluloses which are different in acetyl value from each other.

To the solid phase comprising inorganic material not having a hydrophilic group, acetyl cellulose or a mixture of acetyl celluloses which are different in acetyl value from each other is coated thereon, and the coated acetyl cellulose and a mixture of acetyl celluloses which are different in acetyl value from each other can be saponified. In this case, the surface saponification degree in a range of 5% or more and 100% or less is preferred. It is more preferred to have the surface saponification degree in a range of 10% or more and 100% or less.

Examples of the solid phase comprising inorganic material not having a hydrophilic group including aluminum and the like metals, glass, cement, pottery and the like ceramics, or a porous membrane fabricated by stepping new ceramics, silicon, active charcoal, etc.

With regard to the solid phase of inorganic material having no hydrophilic group, a solid phase prepared by processing of metal such as aluminum, glass, cement, ceramic or new ceramics such as porcelain, silicon, active carbon, etc. may be exemplified.

With regard to the aforementioned solid phase, it is preferred to use in a form of filter or membrane because a solution is able to pass through its inside. In that case, it is preferred that the thickness is 10 µm to 500 µm. It is more preferred that the thickness is 50 µm to 250 µm. In view of easy washing, the thinner the thickness, that is better.

With regard to the aforementioned solid phase where a solution is able to pass through the inside thereof, its average pore size is preferably 0.1 µm to 10 µm. More preferably, an average pore size is 1 µm to 5 µm. As a result, it is possible to achieve a sufficient surface area for adsorption of nucleic acid and, in addition, it hardly happens that the pore is clogged. An average pore size of the solid phase where the solution is able to pass through the inside thereof is able to be decided using a bubble point method (ASTM 316-86; JIS 3832).

It is preferred that the aforementioned solid phase where the aforementioned solution is able to pass the inside thereof is a porous membrane in which the two sides are asymmetric. Here, the expression reading that the two sides are asymmetric shows a property that physical property or chemical property changes from one side of a porous membrane to another. An example of the physical property of the membrane is an average pore size. An example of the chemical property of the membrane is degree of saponification. When a porous membrane in which the two sides are asymmetric is used in the present invention, it is preferred that an average pore size changes from big to small in the direction of passing of the liquid. Here, it is preferred to use a porous membrane in which the ratio of the biggest pore size to the smallest pore size is 2 or more. More preferably, the ratio of the biggest pore size to the smallest pore size is 5 or more. As a result, a sufficient surface area for adsorption of nucleic acid is able to be achieved and, in addition, clogging of the pore hardly takes place.

The nucleic acid-adsorbing solid phase capable of passing a solution through the inside of the membrane having the percentage of porosity in a range of 50 to 95% is preferred. More preferable percentage of porosity is in a range of 65 to 80%. Further, having a bubble point in a range of 0.1 to 10 kgf/cm² is preferred. More preferable bubble point is in a range of 0.2 to 4 kgf/cm².

The nucleic acid-adsorbing solid phase capable of passing a solution through the inside of the membrane having a pressure loss in a range of 0.1 to 100 kPa is preferred. As a result, a uniformed pressure can be obtained at pressurized states. More preferable pressure loss is in a range of 0.5 to 50 kPa. Herein, the term "pressure loss" represents the minimum pressure necessary for passing water through per 100 µm thickness of a membrane.

The nucleic acid-adsorbing solid phase capable of passing a solution through the inside of the membrane having an amount of water percolation, at the time of passing water through under 1 kg/cm² pressure at 25°C, in a range of 1 to 5000 mL per 1 cm² membrane for 1 minute is preferred. More preferable amount of water percolation, at the time of passing water through under 1 kg/cm² pressure at 25°C, is in a range of 5 to 1000 mL per 1 cm² membrane for 1 minute.

The nucleic acid-adsorbing solid phase capable of passing a solution through the inside of the membrane having an amount of nucleic acid-adsorption of 0.1 µg or more per 1 mg of a porous membrane is preferred. More preferable amount of nucleic acid-adsorption is 0.9 µg or more per 1 mg of a porous membrane.

When passing a nucleic acid mixture solution through a nucleic acid-adsorbing porous membrane, it is preferred to have the flow rate in a range of 2 to 1500 µL/sec per unit area cm² of the membrane to obtain suitable contact time of the solution to the porous membrane. When the contact time of the solution to the porous membrane is too short, sufficient separation and purification effect cannot be obtained, and when too long, it is not preferred due to its operability. The flow rate in a range of 5 to 700µL/sec per unit area cm² of the membrane is preferred.

In addition, the nucleic acid-adsorbing porous membrane capable of passing a solution through the inside of the membrane can be used in one layer, but also can be used in multi-layers. The multi-layers of the nucleic acid-adsorbing porous membrane can be identical to or different from each other.

When nucleic acid is adsorbed with the solid phase and then the solid phase is washed, recovered amount and purity of nucleic acid are enhanced and the necessary amount of a sample containing nucleic acid is able to be made very little. When washing and recovering operations are automated, the operations are able to be carried out easily and quickly. The washing operation may be done only once for making the operation quick while, purity is more important, it is preferred to wash for plural times.

In the washing step, the washing solution is a solution containing at least one of water-soluble organic solutions and water-soluble salts is preferred. It is necessary for a washing solution to have ability that works to wash out impurities of the nucleic acid mixture solution, which are adsorbed onto the nucleic acid-adsorbing solid phase along with nucleic acid. In this regard, the washing solution must have such a composition that it desorbs only impurities from the nucleic acid-adsorbing solid phase, and not the nucleic acid. In the purpose, nucleic acid are very insoluble to water-soluble organic solvents such as alcohol, therefore the water-soluble organic solvent is suitable for desorbing other substances by maintaining nucleic acid. In addition, adding water-soluble salts enables to increase an adsorption effect of nucleic acid, thereby improving the selectively removing operation for impurities and unnecessary substances.

With regard to a water-soluble organic solvent to be contained in a washing solution, methanol, ethanol, isopropanol, n-propanol, butanol, acetone, etc. may be used and, among them, it is preferred to use ethanol. Amount of the water-soluble organic solvent contained in the washing solution is preferably 20 to 100% by weight and, more preferably, 40 to 80% by weight.

On the other hand, for the water-soluble salt contained in a washing solution, a halide salt is preferred and among them, a chloride salt is more preferred. Further, the water-soluble salt is preferably a monovalent or divalent cation, particularly an alkali metal and an alkali earth metal is preferred. And among them, a sodium salt, lithium salt and a potassium salt are most preferred, and a sodium salt is particularly preferred. When the water-soluble salt is contained in the washing solution, the concentration thereof is preferable 10 mmol/L or more, and the upper limit is not particularly limited as long as the upper limit does not affect solubility of the impurities, 1 mol/L or less is preferred and 0.1 mol/L or less is more preferred. Above all, that the water-soluble salt is sodium chloride and sodium chloride is contained in 20 mmol/L or more is particularly preferred.

Although the pH of the washing solution may be 3 to 11, it is able to be selected preferably from pH 5 to 10 and, more preferably, from pH 6.5 to 8.5.

In addition, the washing solution is characterized in that a chaotropic substance is not contained therein. As a result, a possibility of having the chaotropic substance incorporated into a recovery step after the washing step can be reduced. In the recovery step, where the chaotropic substance is incorporated thereinto, it sometimes hinders an enzyme reaction such a PCR reaction or the like, therefore considering the afterward enzyme reaction, not including the chaotropic substance to a washing solution is ideal. Further, the chaotropic substance is corrosive and harmful, in this regard, it is extremely advantageous from an operational safety standpoint for the researcher not to use the chaotropic substance when unnecessary.

Herein, the chaotropic substance represents aforementioned urea, guanidine chloride, guanidine isothiocyanate, guanidine thiocyanate, sodium isothiocyanate, sodium iodide, potassium iodide, etc.

Since no chaotropic salt is contained, measured value of the spectrophotometer at 260/230 nm shows a value of more than 1.5 whereby nucleic acid of high purity is able to be purified. Thus, the purified nucleic acid is able to be subjected to experiments with high precision without causing any inconvenience in conducting a molecular biological experiment such as PCR.

Since the washing solution has high wettability for a cartridge or the like container, the washing solution sometimes remains in the container during the washing step in the nucleic acid separation purification process, so that the recovery step after the washing step is contaminated with the washing solution to cause reduction of the purity of nucleic acid and reduction of the reactivity in the subsequent step. Thus, in the first method and the second method of the present invention, when adsorption and desorption of nucleic acid are carried out using a cartridge or the like container, it is important that a solution to be used in the adsorption or washing, particularly the washing solution, does not remain in the cartridge so that it does not exert influence upon the next step.

Accordingly, in order to prevent contamination of the elution solution of the subsequent step with the washing solution of the washing step and thereby to keep residue of the washing solution in the cartridge to the minimum, it is desirable that surface tension of the washing solution is less than 0.035 J/m². When the surface tension is low, wettability of the washing solution for the cartridge is improved and volume of the residual solution can be controlled.

However, the ratio of water can be increased in order to increase the washing efficiency, but in that case, surface tension of the washing solution is increased and amount of the residual solution is increased. When surface tension of the washing solution is 0.035 J/m² or more, amount of the residual solution can be controlled by increasing water repellency of the cartridge. By increasing water repellency of the cartridge, droplets are formed, and amount of the residual solution can be controlled by flow down of the droplets. Examples of the method for increasing water repellency include coating of a water repellant such as silicon on the cartridge surface, kneading of a water repellant such as silicon at the time of the cartridge forming, and the like, though not limited thereto.

In a washing procedure, the amount of a washing solution is preferably 2 µl/mm² or more. When large quantity of the washing solution is used, the washing effect could improve, but in order to maintain the operationability and prohibit the sample from discharging, 200 µl/mm² or less is preferred.

In a washing procedure, when passing a washing solution through a nucleic acid-adsorbing porous membrane, it is preferred to have the flow rate in a range of 2 to 1500 µl/sec per unit area (cm²) of the membrane, and more preferably in a range of 5 to 700 µl/sec. Normally, the passing speed is reduced to elongate the time so that washing is sufficiently conducted. However, preferably, by using the aforementioned range in the invention the step for separating and purifying RNA can be conducted rapidly without reducing the washing efficiency.

In the washing step, a temperature of the washing solution in a range of 4 to 70°C is preferred. Further, a temperature of the washing solution at room temperature is more preferred. In addition to the washing step, stirring using an ultrasonic or a mechanical vibration can be applied to the cartridge for separation and purification of nucleic acid at the same time. On the other hand, washing can be done by conducting a centrifugation.

Amount of the washing solution which is able to be injected at one time may be freely selected within a range of volume of a cartridge. For example, when the cartridge volume is 800 µl, the amount may be within a range of 50 to 800 µl. Preferably, it may be 400 to 800 µl.

With regard to the total amount of the washing solution may be from 1/4 to 10-fold of the sample solution injected into the cartridge. Preferably, it may be selected from the range of 1/2 to 4-fold volume.

Before the aforementioned washing step or during said step, an RNase solution is contacted to the solid phase whereby RNA is previously able to be decomposed when the aimed nucleic acid to be recovered is DNA. When the aimed nucleic acid is RNA, a DNase solution is contacted to the solid phase whereby DAN is previously able to be decomposed. In any of those cases, it is important that the solid phase is washed with a washing solution after that whereby the RNase or the DNase is removed from the solid phase.

Then the aforementioned solid phase after washing is contacted to a solvent which is able to desorb nucleic acid which is adsorbed with the solid phase. Since the solution contains the aimed nucleic acid, it is recovered and provided to amplification of nucleic acid in the succeeding operation such as a PCR (polymerase chain reaction).

Volume of a elution solution to volume of the sample solution containing nucleic acid prepared from a test body is adjusted whereby desorption of nucleic acid is able to be carried out. Volume of the recovered solution containing nucleic acid which is separated and purified is dependent upon the amount of the test body used at that time. Although the commonly used amount of the recovered solution is from several tens to several hundred µl, that may be changed within a range of 1 µl to several tens ml when the sample amount is very little or, reversely, a large amount of nucleic acid is to be separated and purified.

For the elution solution, purified distilled water, Tris buffer, Tris/EDTA buffer and the like can preferably be used. It is preferred that pH of a elution solution is 2 to 11 and, more preferably, 5 to 9. In addition, ionic strength and salt concentration particularly affect the elution of adsorbed nucleic acid. Preferably, the elution solution has an ionic strength of 290 mmol/L or less and has a salt concentration of 90 mmol/L or less, preferably 20 mmol/L or less. As a result thereof, recovering rate of nucleic acid increases and much more nucleic acid is able to be recovered.

Temperature of the elution solution is able to be made 5°C to 90°C. Preferably, it may be 10°C to 50°C or, more preferably, it may be 15°C to 35°C.

When volume of a elution solution is made small as compared with the initial volume of a sample solution containing nucleic acid, it is now possible to prepare a recovered solution containing concentrated nucleic acid. Preferably, the ratio of (volume of elution solution):(volume of sample solution) is able to be made 1:100 to 99:100 and, more preferably, it is able to be made 1:10 to 9:10. As a result thereof, nucleic acid is now able to be easily concentrated without conducting an operation for concentrating in a step after separation and purification of nucleic acid. According to such a method, a method for producing a nucleic acid solution in which nucleic acid is concentrated as compared with a test body is able to be provided.

There is no limitation for the infusing times for a elution solution and that may be either once or plural times. Usually, when nucleic acid is to be separated and purified quickly and simply, that is carried out by means of one recovery while, when a large amount of nucleic acid is to be recovered, elution solution may be infused for several times.

Also, in the recovering step, it is possible to add a stabilizing agent for preventing degradation of nucleic acid recovered in the elution solution of nucleic acid. As the stabilizing agent, an antibacterial agent, a fungicide, a nucleic acid degradation inhibitor and the like can be added. As the nuclease inhibitor, EDTA and the like can be cited. In addition, as another embodiment, a stabilizer can also be added to the recovery container in advance.

It is preferred that the unit for separation and purification of nucleic acid used in the present invention includes (a) a solid phase, (b) a container having at least two openings which receives the aforementioned solid phase and (c) an apparatus for generation of pressure difference connected to one of the openings of the aforementioned container. Hereinafter, an illustration will be made for this unit for separation and purification of nucleic acid.

There is no particular limitation for the material of the container and, although anything may be used so far as it is able to receive the solid phase and at least two openings are able to be formed, plastic is preferred in view of easiness in its manufacture. It is preferred to use a transparent or nontransparent resin such as polystyrene, polymethacrylate, polyethylene, polypropylene, polyester, Nylon and polycarbonate.

Any container may be acceptable provided that it has a receiving part for a solid phase, a solid phase is able to receive the receiving part, the solid part does not come outside the receiving part during suction and discharge of the sample solution, etc. and the opening is able to be connected to a device for generation of pressure difference such as an injector. For such a purpose, it is preferred that the container is divided into two parts in initial stage and, after it receives the solid phase, it is able to be united. In order to prevent that the solid phase comes out from the receiving part, mesh prepared by a material which does not pollute nucleic acid may be placed on upside and downside of the solid phase.

There is also no particular limitation for the shape of the solid phase received in the aforementioned container and, although any form such as circle, square, rectangle and ellipse is acceptable or, when it is a membrane, form such as tube, roll and beads coated with an organic macromolecule having hydroxyl group on the surface is acceptable, it is preferred, in view of adaptability for the manufacture, to be a highly symmetric shape such as circle, square, tube or roll and beads where the surface thereof is coated with an organic macromolecule having hydroxyl group.

The container is usually prepared in a form being divided into a main body receiving the solid phase and a cover and at least one opening is formed in any of them. The opening is used as an inlet and an outlet for a sample solution containing nucleic acid, a washing solution and a solution which is able to desorb the nucleic acid adsorbed with the solid phase (hereinafter, they will be referred to as "sample solution, etc.") and is connected to an apparatus for generating the pressure difference which makes the pressure in inside of the container high or low. Although there is no particular limitation for the shape of the main body, it is preferred to make the cross section thereof circular so that the sample solution, etc. are easily able to be diffused to whole surface of the solid phase. It is also preferred to make the cross section into a tetragonal form so that offcuts of the solid phase are not generated.

It is necessary the aforementioned cover is connected to the main body so that the apparatus for generation of pressure difference is able to make the pressure of the inside of the container low and high and, so far as such a state is able to be achieved, method for connection may be freely selected. For example, use of adhesive, stuffing-in, inserting, screwing and fusion by ultrasonic heating may be listed.

Inner volume of the container is decided only by the amount of the sample solution to be treated and, usually, it is expressed by the volume of the solid phase to be received. Thus, it is preferred to be a size by which one to about six sheet(s) of the solid phase having thickness of not more than above 1 mm (such as about 50 to 500 µm) and diameter of about 2 mm to 20 mm is/are able to be received.

It is preferred that the end of the solid phase is closely contacted to the inner wall side of the container to such an extent that the sample solution, etc. do not pass therethrough.

Under the solid phase encountering to the opening used as an inlet for the sample solution, etc., space is formed without closely contacting to the inner wall of the container so as to make such a structure that the sample solution, etc. are diffused onto the whole surface of the solid phase as uniformly as possible.

On the solid phase encountering to the opening connected to the apparatus for generation of pressure difference, it is preferred to install a material where a hole is formed nearly at the center. The material has an effect of pushing the solid phase and also of efficiently discharging the sample solution, etc. and it is preferred to be in a shape having a slope such as funnel or a cup so that the liquid is concentrated to the central hole. Size of the hole, angle of the slope and thickness of the material are able to be appropriately decided by persons skilled in the art by taking amounts of the sample solution, etc. to be treated, size of the container where the solid phase is received, etc. into consideration. It is preferred to form a space between the material and said opening for storing the over-flown sample solution, etc. so that suction of them into the apparatus for generation of pressure different is prevented. Size of this space is also able to be appropriately decided by persons skilled in the art. For a purpose of a sufficient collection of nucleic acid, it is preferred to suck the sample solution containing nucleic acid in an amount which is not less than the amount by which all solid phase is dipped.

It is also preferred to form a space between the solid phase and the material so that accumulation of the sample solution, etc. only to the part which is immediately beneath the sucking opening is prevented and the sample solution, etc. are able to pass through the inner area of the solid phase relatively uniformly. For such a purpose, it is preferred to form plural projections from said material in the direction of the solid phase. Although size and numbers of the projections are able to be appropriately selected by persons skilled in the art, it is preferred that the opening area of the solid phase is kept as large as possible together with maintaining the space.

When three or more openings are formed in the container, it goes without saying that excessive opening(s) is/are temporally closed so that suction and discharging of the liquid as a result of operations of making the pressure high and low are made possible.

The apparatus for generation of pressure difference firstly makes the inner area of the container wherein the solid phase is received low so that the sample solution containing nucleic acid is sucked. Examples of the apparatus for generation of pressure difference are injector, pipettor and pump such as peristaltic pump where suction and compression are possible. Among them, injector is suitable for manual operation while pump is suitable for automated operation. Pipettor has an advantage that operation by one hand is able to be easily conducted. It is preferred that the apparatus for generation of pressure difference is connected to one of the openings of the aforementioned container in a freely detachable manner.

Now, a method for purification of nucleic acid using the aforementioned unit for separation and purification of nucleic acid will be illustrated.

In a method for separation and purification of nucleic acid according to the present invention, adsorption and desorption of nucleic acid is able to be conducted using a unit for separation and purification of nucleic acid where the aforementioned solid phase is received in a container having at least two openings.

More preferably, adsorption and desorption of nucleic acid are able to be carried out using a unit for separation and purification of nucleic acid which contains (a) a solid phase, (b) a container having at least two openings which receives the aforementioned solid phase and (c) an apparatus for generation of pressure difference being connected to one of the openings of the aforementioned container.

In that case, the first embodiment of the method for separation and purification of nucleic acid according to the present invention may contain the following steps. Thus,
(a) a step where a pretreatment solution is added to the aforementioned sample solution containing nucleic acid so that nucleic acid is adsorbed with the solid phase,
(b) a step where one of the openings of the unit for separation and purification of nucleic acid is inserted into the aforementioned solution containing nucleic acid for adsorbing to solid phase,
(c) a step where inside of the container is made in a state of reduced pressure using an apparatus for generation of pressure difference connected to another opening of the unit for separation and purification of nucleic acid so that a solution containing nucleic acid for adsorbing to the solid phase is sucked and contacted to the solid phase,
(d) a step where inside of the container is made in a compressed state using an apparatus for generation of pressure difference connected to another opening of the unit for separation and purification of nucleic acid so that a solution containing nucleic acid for adsorbing to solid phase is discharged to the outside of the container,
(e) a step where one of the openings of the unit for separation and purification of nucleic acid is inserted into a washing solution,
(f) a step where the inside of the container is made in a state of reduced pressure using an apparatus for generation of pressure difference connected to another opening of the unit for separation and purification of nucleic acid so that the washing solution is sucked and contacted to the solid phase,
(g) a step where the inside of the container is made in a pressurized state using an apparatus for generation of pressure difference connected to another opening of the unit for separation and purification of nucleic acid so that the sucked washing solution is discharged to the outside of the container,
(h) a step where one of the openings of the unit for separation and purification of nucleic acid is inserted into a elution solution where nucleic acid adsorbed with the solid phase is able to be desorbed,
(i) a step where the inside of the container is made in a state of reduced pressure using an apparatus for generation of pressure difference connected to another opening of the unit for separation and purification of nucleic acid so that a solution where the nucleic acid adsorbed with the solid phase is able to be desorbed, is sucked and contacted to the solid phase; and
(j) a step where the inside of the container is made into a pressurized state using an apparatus for generation of pressure difference connected to another opening of the unit for separation and purification of nucleic acid so that an elution solution where the nucleic acid adsorbed with the solid phase is able to be desorbed is discharged to the outside of the container.

It is preferred that, during the steps (c), (f) and (i), a solution in an amount of contacting to nearly all of the solid phase is able to be sucked. However, the apparatus is polluted when suction is conducted to the inside of the apparatus for generation of pressure difference and, therefore, that is adjusted to be an appropriate amount. After suction of an appropriate amount of the solution, inside of the container of the unit for separation and purification of nucleic acid is pressurized and the sucked solution is discharged. It is not necessary to have an intermission until such an operation but discharging may be conducted immediately after the suction.

The second embodiment of a method for separation and purification of nucleic acid according to the present invention may contain the following steps.
(a) a step where a pretreatment solution is added to a sample solution containing nucleic acid to prepare a solution containing nucleic acid for adsorbing to a solid phase,
(b) a step where a solution containing nucleic acid for adsorbing to solid phase is injected into one of the openings of the unit for separation and purification of nucleic acid,
(c) a step where the inside of the container is made into a pressurized state using an apparatus for generation of pressure difference attached to one of the aforementioned openings of the unit for separation and purification of nucleic acid and a solution containing nucleic acid for adsorbing to solid phase injected thereinto is discharged from another opening so that it is contacted to the solid phase whereupon nucleic acid is adsorbed to the solid phase,
(d) a step where a washing solution is injected into the aforementioned one of the openings of the unit for separation and purification of nucleic acid,
(e) a step where the inside of the container is made into a pressurized state using an apparatus for generation of pressure difference attached to one of the aforementioned openings of the unit for separation and purification of nucleic acid and the washing solution injected thereinto is discharged from the aforementioned another opening so that it is contacted to the solid phase whereupon the solid phase is washed,
(f) a step where a solution which is able to desorb the nucleic acid adsorbed with the solid phase is injected into the aforementioned another opening of the unit for separation and purification of nucleic acid and
(g) a step where the inside of the container is made into a pressurized state using an apparatus for generation of pressure difference attached to one of the aforementioned openings of the unit for separation and purification of nucleic acid and the solution which is able to desorb nucleic acid injected thereinto is discharged from the aforementioned another opening so that nucleic acid adsorbed with the solid phase is desorbed and discharged to the outside of the container.

Firstly, one of the openings of the aforementioned unit for separation and purification of nucleic acid is inserted into a sample solution containing nucleic acid.

Although there is no limitation for the addition of the sample solution to a container in the aforementioned step, it is preferred to use a device for experiments such as pipette and syringe. It is more preferred that such a device is nuclease-free or pyrogen-free.

There is no particular limitation for a method of mixing the sample. Upon mixing, it is preferred for example to mix for 1 second to 3 minutes at 30 to 3,000 rpm using a stirring device. As a result, yield of nucleic acid to be separated and purified is able to be increased. It is also preferred to mix by conducting a tumbling mixing for 5 to 30 times. It is also possible to mix by conducting a pipetting operation for 10 to 50 times.

As hereunder, there is shown an example where a step of separation and purification of nucleic acid from the sample containing nucleic acid using an apparatus for generation of pressure and a cartridge for separation and purification of nucleic acid where the aforementioned solid phase is received in a container having at least two openings is conducted by an automated manner although the automated apparatus is not limited thereto.

The automated apparatus is an apparatus for separation and purification of nucleic acid in which a solution where separating and purifying operations comprising the following that nucleic acid is adsorbed with a solid phase is injected into said cartridge for separation and purification of nucleic acid where the solution is able to pass the inside thereof using a cartridge for separation and purification of nucleic acid wherein a solid phase adsorbing the nucleic acid is received followed by pressurizing so that nucleic acid in said sample solution is adsorbed with the aforementioned solid phase, then a washing solution is injected into the aforementioned cartridge for separation and purification of nucleic acid and pressurized to remove impurities and nucleic acid adsorbed with the solid phase after injection of the recovered solution is desorbed in the aforementioned cartridge for separation and purification of nucleic acid and recovered together with the recovered solution is carried out in an automated manner. Such an automated apparatus is characterized in being equipped with an installment mechanism having the aforementioned cartridge for separation and purification of nucleic acid, a container for waste liquids receiving the aforementioned sample solution and the discharged solution which is a washing solution, a mechanism for supplying the compressed air by which compressed air is introduced into the aforementioned cartridge for separation and purification of nucleic acid and a dispensing mechanism where the washing solution and the recovered solution are dispensed to the aforementioned cartridge for separation and purification of nucleic acid.

It is appropriate that the aforementioned installment mechanism is equipped with a stand which is installed in the main body of the apparatus, a cartridge holder which is supported to said stand in a freely movable manner in upward and downward directions and holds the aforementioned cartridge for separation and purification of nucleic acid and a container holder which holds the aforementioned waste liquid container and the aforementioned recovering container beneath said cartridge in such a manner that its position to the aforementioned cartridge for separation and purification of nucleic acid is able to be exchanged.

It is appropriate that the aforementioned mechanism for supplying the compressed air is equipped with an air nozzle where compressed air is spouted from the lower end, a compressing head which supports said air nozzle and makes the aforementioned air nozzle move up and down to the aforementioned cartridge for separation and purification of nucleic acid held at the aforementioned cartridge holder and a positioning means which is installed in said compressing head and positions the cartridge for separation and purification of nucleic acid at the rack of the aforementioned installment mechanism.

It is appropriate that the aforementioned dispensing mechanism is equipped with a dispensing nozzle for a washing solution where the aforementioned washing solution is dispensed, a dispensing nozzle for a recovered solution where the aforementioned recovered solution is dispensed, a nozzle moving stand which holds the aforementioned dispensing nozzle for the washing solution and the aforementioned dispensing nozzle for the recovered solution and is able to successively move on the cartridge for separation and purification of nucleic acid held by the aforementioned installment mechanism, a pump for providing a washing solution which sucks a washing solution from a washing solution bottle receiving a washing solution and supplies to the aforementioned dispensing nozzle for a washing solution and a pump for providing a recovered solution which sucks the recovered solution from the bottle for a elution solution where the elution solution is received and supplied to the aforementioned nozzle for dispensing the elution solution.

In accordance with the automated apparatus as mentioned above, it is now possible to compactly constitute a mechanism which is equipped with a cartridge for separation and purification of nucleic acid, an installment mechanism holding a waste liquid container and a recovering container, a mechanism providing compressed air where compressed air is introduced into a cartridge for separation and purification of nucleic acid and a dispensing mechanism where a washing solution and a elution solution are dispensed into the cartridge for separation and purification of nucleic acid whereby the steps for separation and purification of nucleic acid comprising that a solution containing nucleic acid for adsorbing to a solid phase is injected into a cartridge for separation and purification of nucleic acid equipped with the aforementioned solid phase material followed by compressing so that nucleic acid is adsorbed with said solid phase material, then a washing solution is dispensed thereinto so that impurities are washed and discharged and a elution solution is dispensed so that nucleic acid adsorbed with said solid phase membrane material is separated and recovered are automatically carried out whereupon nucleic acid in a sample solution is automatically separated and purified efficiently within short time.

When the aforementioned installment mechanism is constituted in such a manner that of being equipped with a stand, a cartridge holder which holds a cartridge for separation and purification of nucleic acid and is able to move up and down and a container holder which holds a waste liquid container and a recovering container in an exchangeable manner, it is now possible to easily exchange the cartridge for separation and purification of nucleic acid and a set for both containers as well as a waste liquid container and a recovering container.

When the aforementioned mechanism for providing compressed air is constituted in such a manner of being equipped with an air nozzle, a compression head which moves said air nozzle up and down and a positioning means which positions the cartridge for separation and purification of nucleic acid, it now possible to provide the compressed air in a sure manner by a simple mechanism.

When the aforementioned dispensing mechanism is constituted in such a manner of being equipped with a nozzle for dispensing a washing solution, a nozzle for dispensing a recovered solution, a nozzle-moving stand which is successively able to move on the cartridge for separation and purification of nucleic acid, a pump for providing a washing solution which sucks a washing solution from a bottle for a washing solution and supplies it to a dispensing nozzle for a washing solution and a pump for supplying a elution solution which sucks the elution solution from a elution solution bottle and provides to a nozzle for dispensing a elution solution, it is now possible to successively dispense the washing solution and the elution solution by a simple mechanism.

The present invention will now be illustrated in more detail by way of the following Examples although the present invention is not limited thereto.

### Example 1

(1) Preparation of a cartridge unit for separation and purification of nucleic acid
A cartridge unit for separation and purification of nucleic acid having a part which receives a solid phase of porous membrane where inner diameter is 7 mm was prepared using high-impact polystyrene.
(2) As to the porous membrane, a porous membrane where a porous membrane of triacetylcellulose is saponified (pore size: 2.5 µm; diameter: 7 mm; thickness: 100 µm; degree of saponification: 95%) was used and received in a part for receiving a solid phase in the cartridge for separation and purification of nucleic acid prepared in the above (1).
(3) Preparation of a solution for dissolving the tissues, a pretreatment solution and a washing solution
A solution for dissolving the tissues for separation and purification of DNA from the tissue, a pretreatment solution, a washing solution and a elution solution having the formulations shown in Table 1 were prepared.

**[Table 1]**

| [Dissolving Solution for Tissues (for Separation and Purification of DNA from Tissues)] | | |
|---|---|---|
| 1 mol/l Tris hydrochloride (Wako Pure Chemical) | | 26 g |
| Sodium chloride | | 3 g |
| 0.5 mol/l EDTA (Wako Pure Chemical) | | 140 g |
| 10% SDS (Wako Pure Chemical) | | 160 g |
| Distilled Water | | 350 g |
| pH 8.2 | | |
| | | |

| [Pretreatment Solution (for Separation and Purification of DNA from Tissues)] | | |
|---|---|---|
| Guanidine hydrochloride (Wako Pure Chemical) | | 380 g |
| Tween 20 (ICN) | | 120 g |
| Acetylene glycol (Air Products) | | 3 g |
| Silicone oil (GE Toshiba Silicone) | | 0.6 g |
| Bis-Tris | | 15.5 g |
| Distilled water | | 275 g |
| pH 6.0 | | |
| | | |

| [Washing Solution (for Separation and Purification of DNA from Tissues)] | | |
|---|---|---|
| 1 mol/l Tris hydrochloride (Wako Pure Chemical) | | 8 g |
| Sodium chloride (Wako Pure Chemical) | | 4.5 g |
| Ethanol (Wako Pure Chemical) | | 360 g |
| Distilled water | | 350 g |
| pH 7.6 | | |
| | | |

| [Elution solution (for Separation and Purification of DNA from Tissues)] | | |
|---|---|---|
| 1 mol/l Tris hydrochloride (Wako Pure Chemical) | | 2.5 g |
| Distilled water | | 250 g |
| pH 9.0 | | |

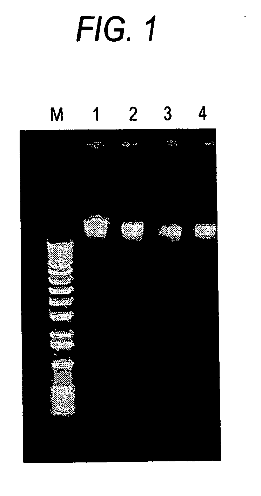

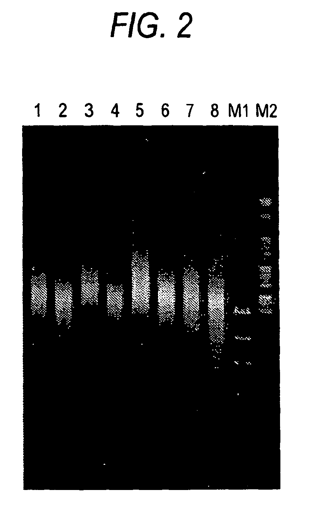

(4) Dissolving treatment for animal cells
Each 5 mg of lung, kidney, tail and liver of a mouse were prepared. Each of those animal tissues was frozen and pulverized by a hammer to prepare small pieces. The small pieces (5 mg) of animal tissue were placed in 1.5-ml nuclease-free and pyrogen-free microtube (platinum tube; manufactured by BM Kiki), then 180 µl of the tissue-dissolved solution prepared in the above (3) and 20 µl of 20 mg/ml protease K (manufactured by Sigma) which was a protease were added thereto and the mixture was stirred and incubated at 55°C for 12 hours to dissolve the animal tissue. Undissolved residual tissue was precipitated by centrifugal separation and the supernatant liquid thereof was placed in another 1.5-ml nuclease-free and pyrogen-free microtube (platinum tube; manufactured by BM Kiki). After that, 20 µl of a 15 mmol/l NaCl solution of 10 mmol/l Tris buffer of 100 mmol/ml RNase (ribonuclease A; manufactured by Sigma) were added thereto followed by incubating at 25°C for 2 minutes.
(5) Separation and purification of nucleic acid
To each of the solutions prepared in the above (4) was added 180 µl of the pretreatment solution prepared in the above (3) and the mixture was stirred and incubated at 70°C for 10 minutes. Then 240 µl of ethanol was added thereto followed by stirring. After that, each of the resulting solutions was injected into one of the openings of a cartridge for separation and purification of nucleic acid having a solid phase of porous membrane prepared in the above (2), then an apparatus for generation of pressure difference (tubing pump) was connected to said one of the openings, the inside of the cartridge for separation and purification of nucleic acid was made into a pressurized state (80 kpa) and the solution injected therein was passed through the solid phase of porous membrane so as to contact to the solid phase of porous membrane and was discharged from another opening of the cartridge for separation and purification of nucleic acid. After that, 750 µl of a washing solution prepared in the above (3) was injected into the aforementioned one of openings of the aforementioned cartridge for separation and purification of nucleic acid, a tubing pump was connected to the above one of the openings, the inside of the cartridge for separation and purification of nucleic acid is made into a compressed state (80 kpa) and the washing solution injected therein was passed through the solid phase of porous membrane and discharged from another opening. That operation was carried out for three times. Then 200 µl of a elution solution prepared in the above (3) was injected into the aforementioned one of the openings of the aforementioned cartridge for separation and purification of nucleic acid, a tubing pump was connected to the aforementioned one of the openings of the cartridge for separation and purification of nucleic acid, the inside of the cartridge for separation and purification of nucleic acid was made into a compressed state (80 kpa) and the elution solution injected thereinto was passed through the solid phase of porous membrane and discharged from another opening to recover this solution. Time required for this operation of separation and purification of nucleic acid (from the sample solution containing nucleic acid was injected into the aforementioned cartridge until it was recovered) was 12 minutes.
(5) Quantification of recovered amount of nucleic acid
With regard to each of the recovered solutions which were recovered in the above Example, the result of electrophoresis of DNA is shown in Fig. 1. Fig. 2 shows the result of electrophoresis of pulse field electrophoresis. Yield of DNA calculated from UV measurement of each recovered solution and ratio of absorbances at 260 nm to 280 nm (A260/A280) showing contamination of protein as an index for the purity are shown in Table 2. Ratio of absorbances at 260 nm to 230 nm (A260/A230) showing contamination of foreign substances as an index for the purity is shown in Table 3. In both of the above, purity is able to be judged to be low when the value is low. As a comparative example, a result by a centrifugal method using a common glass filer (such as DNeasy Tissue Kit; catalog # 69504; Qiagen) is also shown.
The symbols in Fig. 1 represent as follows.
M: Marker for molecular weight
1: Example for lung
2: Example for kidney
3: Example for tail
4: Example for liver
The symbols in Fig. 2 represent as follows.
M1, 2: Markers for molecular weight
1: Example for tail
2: Comparative Example for tail
3: Example for liver
4: Comparative Example for liver
5: Example for lung
6: Comparative Example for lung
7: Example for kidney
8: Comparative Example for kidney

**[Table 2]**

| | Lung | Kidney | Tail | Liver |
|---|---|---|---|---|
| Yield of DNA(µg) | 5.2 | 4.3 | 3.6 | 3.8 |
| A260/A280 | 1.95 | 1.94 | 1.94 | 1.96 |

**[Table 3]**

| Example | Lung | Kidney | Tail | Liver |
|---|---|---|---|---|
| A260/A230 | 1.95-2.29 | 1.94-2.13 | 1.85-1.99 | 1.18-1.32 |

| Comp. Ex. | Lung | Kidney | Tail | Liver |
|---|---|---|---|---|
| A260/A230 | 0.98-2.16 | 1.54-2.01 | 1.32-1.95 | 0.87-1.22 |

As a result of the aforementioned Example, it will be apparent from the results of electrophoresis of Fig. 1 and Fig. 2 that, in the present Example, genomic DNA of high molecular weight is able to be purified and that, as a result of comparison with a centrifugal method using a glass filter in Comparative Example, genomic DNA having clearly higher molecular weight is able be prepared as compared with a centrifugal column method using a common glass filter. In addition, as will be apparent from the result of Table 2 and Table 3, it is noted that, when the method of the present invention is used, nucleic acid is able to be separated and purified from animal tissues quite efficiently. Moreover, as a result of comparison with a centrifugal method using a common glass filter in Comparative Example, it is noted that highly pure DNA is able to be purified where contamination of foreign substances of 230 nm is clearly small. Thus, in accordance with the method of the present invention in which separating property is excellent and washing efficiency is good, nucleic acid was able to be prepared within the aforementioned time quickly and in high yield and high purity.

### [Example 2]

(1) Cartridge for separation and purification of nucleic acid was prepared by the same manner as in Example 1.
(2) Dissolving treatment for animal cells
Each 5 mg of lung, kidney, tail and liver of a mouse were prepared. Each of those animal tissues was frozen and pulverized by a hammer to prepare small pieces. The small pieces (5 mg) of animal tissue were placed in 1.5-ml nuclease-free and pyrogen-free microtube (platinum tube; manufactured by BM Kiki), then 180 µl of ATL solution manufactured by Qiagen and 20 µl of 20 mg/ml protease K (manufactured by Qiagen) which was a protease were added thereto and the mixture was stirred and incubated at 55°C for 12 hours to dissolve the animal tissue. Undissolved residual tissue was precipitated by centrifugal separation and the supernatant liquid thereof was placed in another 1.5-ml microtube (platinum tube; manufactured by BM Kiki) which was nuclease-free and pyrogen-free. After that, 4 µl of a 100 mmol/l RNase (manufactured by Qiagen) solution were added thereto followed by incubating at 25°C for 2 minutes.
(3) Separation and purification of nucleic acid
To each of the solutions prepared in the above (2) was added 200 µl of an AL solution manufactured by Qiagen and the mixture was stirred and incubated at 70°C for 10 minutes. Then 200 µl of ethanol was added thereto followed by stirring. After that, each of the resulting solutions was injected into one of the openings of a cartridge for separation and purification of nucleic acid having a solid phase of porous membrane prepared in the above (1), then an apparatus for generation of pressure difference (tubing pump) was connected to said one of the openings, the inside of the cartridge for separation and purification of nucleic acid was made into a pressurized state (80 kpa) and the solution injected therein was passed through the solid phase of porous membrane so as to contact to the solid phase of porous membrane and was discharged from another opening of the cartridge for separation and purification of nucleic acid. After that, 500 µl of an AW1 liquid (manufactured by Qiagen) washing solution was injected into the aforementioned one of openings of the aforementioned cartridge for separation and purification of nucleic acid, a tubing pump was connected to the above one of the openings, the inside of the cartridge for separation and purification of nucleic acid was made into a pressurized state (80 kpa) and the washing solution injected therein was passed through the solid phase of porous membrane and discharged from another opening. Then 500 µl of an AW2 liquid (manufactured by Qiagen) washing solution was injected into the aforementioned one of the openings of the aforementioned cartridge for separation and purification of nucleic acid, a tubing pump was connected to the aforementioned one of the openings of the cartridge for separation and purification of nucleic acid, the inside of the cartridge for separation and purification of nucleic acid was made into a pressurized state (80 kpa) and the elution solution injected thereinto was passed through the solid phase or porous membrane and discharged from another opening. After that, 200 µl of an AE liquid (manufactured by Qiagen) elution solution was injected into the aforementioned one of the openings of the aforementioned cartridge for separation and purification of nucleic acid, a tubing pump was connected to the aforementioned one of the openings of the aforementioned cartridge for separation and purification of nucleic acid so that the inside of the cartridge for separation and purification of nucleic acid was made into a pressurized state (80 kpa) and the injected elution solution was passed through a solid phase of porous membrane and discharged from another opening to recover this solution. Time required for this operation of separation and purification of nucleic acid (from the sample solution containing nucleic acid was injected into the aforementioned cartridge until it was recovered) was 12 minutes.
(5) Quantification of recovered amount of nucleic acid
With regard to each of the recovered solutions which were recovered in the above Example 2, yield of DNA calculated from UV measurement of each recovered solution, ratio of absorbances at 260 nm to 280 nm (A260/A280) showing contamination of protein as an index for the purity and ratio of absorbances at 260 nm to 230 nm (A260/A230) showing contamination of foreign substances as an index for the purity are shown in the Tables. In both of the above, purity is able to be judged to be low when the value is low. In this Example 2, nucleic acid with lower purity than Example 1 was recovered.
Although extraction of nucleic acid is possible, the product has a low purity.

**[Table 4]**

| | Lung | Kidney | Tail | Liver |
|---|---|---|---|---|
| Yield of DNA (µg) | 5.0 | 4.1 | 3.0 | 3.2 |
| A260/A280 | 1.95 | 1.94 | 1.94 | 1.96 |

**[Table 5]**

| Example | Lung | Kidney | Tail | Liver |
|---|---|---|---|---|
| A260/A230 | 1.00-1.93 | 1.45-1.90 | 1.30-1.85 | 0.84-1.15 |

### [Example 3]

(1) Cartridge for separation and purification of nucleic acid was prepared by the same manner as in Example 1.
(2) Dissolving treatment for animal cells
Tail (5 mg) of a mouse was prepared. Small pieces (5 mg) of the animal tissue were placed in a 1.5-ml nuclease-free and pyrogen-free microtube (platinum tube; manufactured by BM Kiki), 180 µl of a tissue dissolving solution prepared in Example 1 and 20 µl of a 20 mg/ml solution of protease K (manufactured by Qiagen) which was a protease were added thereto and the mixture was stirred and incubated for 12 hours at 55°C to dissolve the animal tissue. Undissolved residual tissue was precipitated by centrifugal separation and the supernatant liquid thereof was placed in another 1.5-ml nuclease-free and pyrogen-free microtube (platinum tube; manufactured by BM Kiki).
(3) Separation and purification of nucleic acid
A mixture of 180 µl of a pretreatment solution prepared in Example 1 and 240 µl of ethanol was added to each of the solutions prepared in the above (2) and stirred for 15 seconds using a vortex. After that, the solution was injected into one of the openings of the cartridge for separation and purification of nucleic acid having a solid phase of porous membrane prepared in the above (1), then an apparatus for generation of pressure difference (tubing pump) was connected to the aforementioned one of the openings so that the inside of the cartridge for separation and purification of nucleic acid was made in a pressurized state (80 kpa) and the injected solution was passed through the solid phase of porous membrane to contact to the solid phase of porous membrane and discharged from another opening of the cartridge for separation and purification of nucleic acid. After that, 750 µl of a washing solution prepared in Example 1 was injected into the aforementioned one of the openings of the cartridge for separation and purification of nucleic acid, a tubing pump was connected to the above one of the openings, the inside of the cartridge for separation and purification of nucleic acid was made into a pressurized state (80 kpa) and the injected washing solution was passed through the solid phase of porous membrane and discharged from another opening. The operation was conducted for three times. After that, 200 µl of the elution solution prepared in Example 1 was injected into the above one of the openings of the cartridge for separation and purification of nucleic acid, a tubing pump was connected to the above one of the openings of the cartridge for separation and purification of nucleic acid so that the inside of the cartridge for separation and purification of nucleic acid was made into a pressurized state (80 pka) and the injected elution solution was passed through the solid phase of porous membrane, discharged from another opening and recovered. Time required for the operation of separation and purification of nucleic acid (from injection of the nucleic acid-containing sample solution into the aforementioned cartridge until recovering) was 10 minutes.
(5) Quantification of recovered amount of nucleic acid
With regard to each of the recovered solutions which were recovered in the above Example 3, yield of DNA calculated from UV measurement of each recovered solution, ratio of absorbances at 260 nm to 280 nm (A260/A280) showing contamination of protein as an index for the purity and ratio of absorbances at 260 nm to 230 nm (A260/A230) showing contamination of foreign substances as an index for the purity are shown in the Tables. In both of the above, purity is able to be judged to be low when the value is low. In this Example, nucleic acid with similar purity as in Example 1 was recovered.

**[Table 6]**

| | Tail |
|---|---|
| Yield of DNA(µg) | 4.3 |
| A260/A280 | 2.20 |

**[Table 7]**

| Example | Tail |
|---|---|
| A260/A230 | 2.05 |

### [Example 5]

(1) Preparation of a cartridge for purification of nucleic acid
A cartridge for purification of nucleic acid of inner diameter of 7 mm having a part receiving a nucleic acid-adsorptive porous membrane was prepared using high-impact polystyrene.
(2) As to a nucleic acid-adsorptive porous membrane, a porous membrane (pore size: 2.5 µm; diameter: 7 mm; thickness: 100 µm; degree of saponification: 95%) prepared by a saponification treatment of porous membrane made of triacetylcullose was used and received in a part for receiving a nucleic acid-adsorptive porous membrane of the cartridge for separation and purification of nucleic acid prepared in the above (1).
(3) Preparation of an original solution for nucleic acid-solubilizing reagent and a washing solution
An original solution for nucleic acid-solubilizing reagent and a washing solution having the following formulations were prepared.

| (Original solution for nucleic acid-solubilizing reagent 1) adjusted to pH 6.0 | |
|---|---|
| Guanidine hydrochloride (Wako Pure Chemical) | 5 mol/l |
| BisTris (Dojindo Laboratories) | 300 mmol/l |
| POE sorbitan monooleate (Wako Pure Chemical) | 7.5% (v/v) |
| Distilled water | 1,000 ml |
| (Washing solution) 10 mmol/l Tris-HCl (Life Technology) | 50% (v/v) ethanol |
| (Elution solution) 10 mmol/l Tris-HCl (Life Teclmology) | |

(4) Operation of separation and purification of nucleic acid
A solution of protease ("Amano" G which is protease N) (30 µl) and 250 µl of the nucleic acid-solubilizing original solution 1 were added to 200 µl of human whole blood in which heparin Na or EDTA-2Na was used as an anticoagulant and the mixture was incubated at 56°C for 2 minutes. Incidentally, heparin Na and EDTA-2Na were contained in concentrations of 13 units/ml and 1.5 mg/ml, respectively. After the incubation, 250 µl of ethanol was added thereto followed by stirring. After stirring, it was injected into one of the openings of the cartridge for purification of nucleic acid having a nucleic acid-adsorptive porous membrane prepared in the above (2), then an apparatus for generation of pressure difference (tubing pump) was connected to the above one of the openings so that the inside of the cartridge of separation and purification of nucleic acid was made into a pressurized state (80 kpa) and the injected sample solution containing nucleic acid was passed through the nucleic acid-adsorptive porous membrane so as to contact to the nucleic acid-adsorptive porous membrane and discharged from another opening of the cartridge for separation and purification of nucleic acid. After that, a washing solution was injected into the above one of the openings of the above cartridge for separation and purification of nucleic acid, a tubing pump was connected to the above one of the openings so that the inside of the cartridge for separation and purification of nucleic acid was made into a pressurized state (80 kpa) and the injected washing solution was passed through the nucleic acid-adsorptive porous membrane and discharged from another opening. After that, a elution solution was injected into the above one of the openings of the above cartridge for separation and purification of nucleic acid, a tubing pump was connected to the above one of the openings of the cartridge for separation and purification of nucleic acid so that the inside of the cartridge for separation and purification of nucleic acid was made into a pressurized state (80 kpa) and the injected elution solution was passed through the nucleic acid-adsorptive porous membrane, discharged from another opening and recovered.

### [Comparative Example 1]

An original solution for solubilization of nucleic acid having the following formulation was prepared.

| (An original solution for nucleic acid-solubilizing reagent 2) | (pH 5.5) |
|---|---|
| Guanidine hydrochloride (Wako Pure Chemical) | 5 mol/l |
| POE sorbitan monostearate (Kao) | 3% (v/v) |
| Distilled water | 1000 ml |

The same operation as in the above (4) was conduced except that the aforementioned original solution of nucleic acid-solubilizing reagent 2 was used as an original solution of nucleic acid-solubilizing reagent.

### [Comparative Example 2]

An original solution for solubilization of nucleic acid having the following formulation was prepared.

| (An original solution for nucleic acid-solubilizing reagent 3) | adjusted to pH 7.0 |
|---|---|
| Guanidine hydrochloride (Wako Pure Chemical) | 5 mol/l |
| Tris-HCl (Life Technology) (pH 7.0) | 100 mmol/l |
| POE sorbitan monostearate (Wako Pure Chemical) | 7.5% (v/v) |
| Distilled water | 1000 ml |

The same operation as in the above (4) was conduced except that the aforementioned original solution of nucleic acid-solubilizing reagent 3 was used as an original solution of nucleic acid-solubilizing reagent.
(5) Quantification of purity of the recovered nucleic acid
Purity of the recovered nucleic acid was quantified by measurement of absorbance of the aforementioned recovered solution. Result of measurement of absorbance for each wavelength is shown in Table 8.

**[Table 8]**

| | Anticoagulant | Wavelength (nm) | | | | | 260/280 |
|---|---|---|---|---|---|---|---|
| | | 230 | 260 | 280 | 320 | 400 | |
| Example 5 | Heparin Na | 0.355 | 0.631 | 0.340 | 0.020 | 0.014 | 1.909 |
| | EDTA-2Na | 0.291 | 0.542 | 0.287 | 0.013 | 0.008 | 1.931 |
| Comparative Example 1 | Heparin Na | 0.707 | 0.837 | 0.588 | 0.297 | 0.200 | 1.856 |
| | EDTA-2Na | 0.297 | 0.539 | 0.286 | 0.028 | 0.013 | 1.981 |
| Comparative Example 2 | Heparin Na | 0.456 | 0.610 | 0.386 | 0.130 | 0.090 | 1.875 |

When purity of the recovered nucleic acid is low, absorbance at 320 nm or 400 nm becomes high.
As will be apparent from the result of Table 8, when the method of the present invention is used, it is noted that nucleic acid of high purity is able to be very efficiently recovered and purified from blood regardless of the type of the anticoagulant.

### [Example 7]

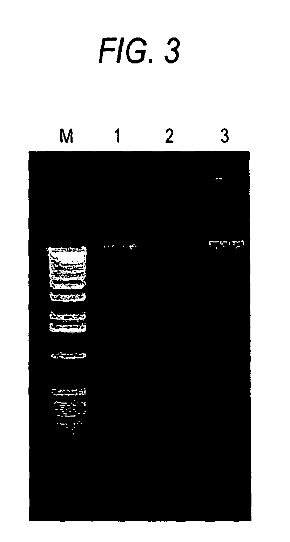

(1) According to the same manner as in Example 1, extraction of genomic DNA from oral swab was carried out using a cartridge prepared by receiving porous membrane, a pretreatment solution, a washing solution and a elution solution.
(2) Treatment of oral swab
Inside of the mouth was scraped off using a swab. PBS (phosphate-equilibrated buffer) (200 µl) was dispensed in a 1.5-ml nuclease-free and pyrogen-free microtube (platinum tube; manufactured by BM Kiki) and the above swab was moved up and down for several times so that bacteria in the mouth were suspended. The pretreatment solution (200 µl) prepared in Example 1 (3) was added thereto and stirred therewith, then 10 µl of a 20 mg/ml solution of Protease K (protease manufactured by Sigma) was added thereto and stirred therewith and the mixture was incubated at 56°C for 10 minutes to dissolve the cells.
(3) Separation and purification of nucleic acid
Ethanol (200 µl) was added to the solution prepared in the above (2) followed by stirring. After that, the solution was injected into one of the openings of the cartridge for separation and purification of nucleic acid having a solid phase of porous membrane prepared in the aforementioned (1), then an apparatus for generation of pressure difference (tubing pump) was connected to the aforementioned one of the openings so that the inside of the cartridge for separation and purification of nucleic acid was made into a pressurized state (80 kpa) and the injected solution was passed through the solid phase of porous membrane so as to contact to the solid phase of porous membrane and discharged from another opening of the cartridge for separation and purification of nucleic acid. After that, a washing solution prepared in Example 1 (3) was injected into the aforementioned one of the openings of the cartridge for separation and purification of nucleic acid, a tubing pump was connected to the aforementioned one of the openings of so that the inside of the cartridge for separation and purification of nucleic acid was made into a pressurized state (80 kpa) and the injected washing solution was passed through the solid phase of porous membrane and discharged from another opening. After that, a elution solution prepared in Example 1 (3) was injected into the aforementioned one of the openings of the aforementioned cartridge for separation and purification of nucleic acid, a tubing pump was connected to the aforementioned one of the openings of the cartridge for separation and purification of nucleic acid so that inside of the cartridge for separation and purification of nucleic acid was made into a pressurized state (80 kpa) and the injected elution solution was passed through the solid phase of porous membrane, discharged from another opening and recovered. Time required for the operation of separation and purification of nucleic acid (from injection of the nucleic acid-containing sample solution into the aforementioned cartridge until recovering) was 2 minutes.
(5) Quantification of recovered amount of nucleic acid
For each of the recovered solutions which were recovered in the aforementioned Example, the result of electrophoresis for DNA is shown in Fig. 3. Yield of DNA calculated from UV measurement of each recovered solution and the ratio of absorbances (A260/A230) at 260 nm and 230 nm showing contamination of foreign substances are shown in Table 9. In both of them, purity is able to be judged to be low when the value is low. As a Comparative Example, the result using a common glass filter (such as DNeasy Tissue Kit; catalog # 69504; Qiagen) and by a fully automate extracting machine using magnetic beads (such as EZ-1 DNA Tissue Kit, catalog # 953034 and EZ-1 DNA Buccal Swab Card, catalog # 9015589); Qiagen) was also shown.
The symbols in Fig. 3 represent as follows.
M: Marker for molecular weight
1: EZ-1, Mouth swab card method (G2 Swab)
2: DNeasy Tissue Kit (PBS Swab; QIA Spin Column)
3: Example 2

**[Table 9]**

| Example | EZ-1 | DNeasy | Example 2 |
|---|---|---|---|
| Yield (µg) | 0.6 | 0.7 | 0.9 |
| A260/A230 | 0.879 | 0.278 | 1.5 |

As a result of the aforementioned Example, it will be apparent from the results of electrophoresis of Fig. 3 that, in the present Example, genomic DNA of high molecular weight is able to be purified and that, as a result of comparison with the Comparative Example, more genomic DNA is able be prepared as compared with a centrifugal column method using a common glass filter. In addition, as will be apparent from the result of Table 4, it is noted that, when the method of the present invention is used, nucleic acid is able to be separated and purified from animal tissues quite efficiently. Moreover, as a result of comparison with a centrifugal method using a common glass filter in the Comparative Example, it is noted that highly pure DNA is able to be purified where contamination of foreign substances of 230 nm is clearly small. Thus, in accordance with the method of the present invention in which separating property is excellent and washing efficiency is good, nucleic acid was able to be prepared within the aforementioned time quickly and in high yield and high purity.

## Claims

1. A method for separation and purification of nucleic acid comprising:
(1) adding a pretreatment solution and further adding a water-soluble organic solvent to a sample solution containing nucleic acid to prepare a solution containing nucleic acid for adsorbing to a solid phase;
(2) passing the solution containing nucleic acid for adsorbing to a solid phase through a solid phase, to adsorb nucleic acid to the solid phase;
(3) passing a washing solution through the solid phase, to wash the solid phase under such a state that nucleic acid is adsorbed; and
(4) passing a elution solution through the solid phase to desorb nucleic acid from the solid phase,
wherein the pretreatment solution contains BisTris (bis(2-hydroxyethyl)iminotris(hydroxymethyl)methane) as a buffer and is a solution containing a surfactant and at least one selected from the group consisting of antifoaming agent, stabilizer for nucleic acid and chaotropic salt, and the solid phase is a solid phase comprising an organic macromolecule having a polysaccharide structure.

2. The method for separation and purification of nucleic acid according to claim 1, wherein the surfactant contained in said pretreatment solution is a nonionic surfactant.

3. The method for separation and purification of nucleic acid according to claim 2, wherein the nonionic surfactant is a surfactant of a polyoxyethylene type.

4. The method for separation and purification of nucleic acid according to claim 3, wherein the surfactant of a polyoxyethylene type is a surfactant of a polyoxyethylene sorbitan type.

5. The method for separation and purification of nucleic acid according to any one of claims 1 to 4, wherein the sample solution containing nucleic acid in the step (1) is a sample solution prepared by adding a tissue lysis solution to animal tissue to subject to a digesting treatment of said animal tissue.

6. The method for separation and purification of nucleic acid according to any one of claims 1 to 4, wherein the sample solution containing nucleic acid in the aforementioned step (1) is a sample solution containing an anticoagulant and at least one of blood and white blood cell.

7. The method for separation and purification of nucleic acid according to claim 5, wherein the tissue lysis solution is a solution containing at least one of compounds selected from the group consisting of surfactant, buffer, stabilizer for nucleic acid, alkali metal halide, chaotropic agent, protease and antifoaming agent.

8. The method for separation and purification of nucleic acid according to any one of claims 1 to 7, wherein the solid phase comprising the organic macromolecule having a polysaccharide structure is a solid phase comprising acetylcellulose.

9. The method for separation and purification of nucleic acid according to claim 8, wherein the solid phase comprising acetylcellulose is a solid phase comprising an organic macromolecule prepared by subjecting acetylcellulose or a mixture of acetylcelluloses having different acetyl values to a saponification treatment.

10. The method for separation and purification of nucleic acid according to claim 9, wherein the saponification rate of the mixture of acetylcelluloses having different acetyl values is 5% or more.

11. The method for separation and purification of nucleic acid according to claim 1, wherein the solid phase comprising an organic macromolecule having a polysaccharide structure is a solid phase comprising a regenerated cellulose.

12. The method for separation and purification of nucleic acid according to any one of claims 1 to 11, wherein the solid phase is a porous membrane.

13. The method for separation and purification of nucleic acid according to claim 12, wherein the porous membrane is a porous membrane in which the front and back sides are asymmetric.

14. The method for separation and purification of nucleic acid according to claim 12, wherein the porous membrane is a porous membrane having an average pore size of 0.1 to 10.0 µm.

15. The method for separation and purification of nucleic acid according to any one of claims 12 to 14, wherein the porous membrane is a porous membrane having a thickness of 10 to 500 µm.

16. The method for separation and purification of nucleic acid according to one of claims 1 to 15, wherein the water-soluble organic solvent includes at least one of methanol, ethanol, propanol and butanol.

17. The method for separation and purification of nucleic acid according to any one of claims 5 to 16, which comprises removing non-dissolved residual tissue components from the sample solution prepared in the step of adding a tissue lysis solution to animal tissue to subject the animal tissue to a digesting treatment.

18. The method for separation and purification of nucleic acid according to any of claims 5 to 17, which comprises adding a solution of RNase to the sample solution prepared in the step of adding a tissue lysis solution to animal tissue to subject animal tissue to a digesting treatment.

19. The method for separation and purification of nucleic acid according to any one of claims 1 to 18, wherein adsorption and desorption of nucleic acid are carried out using a unit for separation and purification of nucleic acid where a solid phase is housed in a container having at least two openings.

20. The method for separation and purification of nucleic acid according to any one of claims 1 to 19, wherein adsorption and desorption of nucleic acid are carried out using a unit for separation and purification of nucleic acid which contains (a) a solid phase, (b) a container having at least two openings, which houses the solid phase and (c) an apparatus for generation of pressure difference being connected to one of the openings of the aforementioned container.

21. The method for separation and purification of nucleic acid according to claim 20, wherein the apparatus for generation of pressure difference is an apparatus for pressurization.

22. The method for separation and purification of nucleic acid according to claim 20, wherein the apparatus for generation of pressure difference is an apparatus for depressurization.

23. The method for separation and purification of nucleic acid according to any one of claims 20 to 22, wherein the apparatus for generation of pressure difference is attached to one of the openings of the container in a freely detachable manner.

24. The method for separation and purification of nucleic acid according to claim 20 or 21, which comprises the following steps.
(a) adding a pretreatment solution to a sample solution containing nucleic acid to prepare a solution containing nucleic acid for adsorbing to a solid phase;
(b) injecting a solution containing nucleic acid for adsorbing to solid phase into one of the openings of the unit for separation and purification of nucleic acid;
(c) making the inside of the container into a pressurized state using an apparatus for generation of pressure difference attached to one of the aforementioned openings of the unit for separation and purification of nucleic acid, and discharging a solution containing nucleic acid for adsorbing to solid phase injected thereinto from another opening, so that the solution is contacted to the solid phase and nucleic acid is adsorbed to the solid phase;
(d) injection a washing solution into the one of the openings of the unit for separation and purification of nucleic acid;
(e) making the inside of the container into a pressurized state using an apparatus for generation of pressure difference attached to one of the aforementioned openings of the unit for separation and purification of nucleic acid, and discharging the washing solution injected thereinto from the another opening, so that the washing solution is contacted to the solid phase and the solid phase is washed;
(f) injecting an elution solution which is able to desrob the nucleic acid adsorbed with the solid phase into the another opening of the unit for separation and purification of nucleic acid; and
(g) making the inside of the container into a pressurized state using an apparatus for generation of pressure difference attached to the one of the openings of the unit for separation and purification of nucleic acid, and discharging the elution solution injected thereinto from the another opening, so that nucleic acid adsorbed with the solid phase is desorbed and discharged to the outside of the container.

25. The method for separation and purification of nucleic acid according to claim 24, which, before the aforementioned step (f), comprises contacting a DNase solution to the solid phase and then washing the solid phase with a washing solution.

26. The method for separation and purification of nucleic acid according to any one of claims 1 to 25, wherein the washing solution is a solution containing 20 to 100% by weight of methanol, ethanol, isopropanol or n-propanol.

27. The method for separation and purification of nucleic acid according to any one of claims 1 to 26, wherein the elution solution is a solution having a salt concentration of 0.5 mol/L or less.

28. A reagent kit for conducting the method mentioned in any one of claims 1 to 27, comprising: (i) an unit for separation and purification of nucleic acid, (ii) protease, (iii) a chaotropic salt, surfactant and BisTris, (iv) a washing solution, (v) a reagent for a elution solution, and (vi) a water-soluble organic solvent

## Patentansprüche

1. Verfahren zur Abtrennung und Reinigung von Nucleinsäure, umfassend:
(1) Zugeben einer Vorbehandlungslösung und außerdem Zugeben eines wasserlöslichen organischen Lösungsmittels zu einer Probenlösung, die Nucleinsäure enthält, unter Herstellung einer Lösung, die Nucleinsäure zum Adsorbieren an einer festen Phase enthält;
(2) Durchleiten der Lösung, die Nucleinsäure zum Adsorbieren an eine feste Phase enthält, durch eine feste Phase, um Nucleinsäure an der festen Phase zu adsorbieren;
(3) Durchleiten einer Waschlösung durch die feste Phase, um die feste Phase in einem solchen Zustand, dass die Nucleinsäure adsorbiert ist, zu waschen und
(4) Durchleiten einer Elutionslösung durch die feste Phase, um Nucleinsäure von der festen Phase zu desorbieren,
wobei die Vorbehandlungslösung BisTris (Bis(2-hydroxyethyl)iminotris(hydroxymethyl)methan) als Puffer enthält und eine Lösung ist, die ein Surfactant und wenigstens eins, ausgewählt aus der Gruppe, bestehend aus Antischaummittel, Stabilisator für Nucleinsäure und chaotropem Salz, enthält und die feste Phase eine feste Phase ist, die ein organisches Makromolekül umfasst, das eine Polysaccharidstruktur hat.

2. Verfahren zur Abtrennung und Reinigung von Nucleinsäure gemäß Anspruch 1, wobei das in der Vorbehandlungslösung enthaltene Surfactant ein nichtionisches Surfactant ist.

3. Verfahren zur Abtrennung und Reinigung von Nucleinsäure gemäß Anspruch 2, wobei das nichtionische Surfactant ein Surfactant des Polyoxyethylen-Typs ist.

4. Verfahren zur Abtrennung und Reinigung von Nucleinsäure gemäß Anspruch 3, wobei das Surfactant eines Polyoxyethylen-Typs ein Surfactant eines Polyoxyethylensorbitan-Typs ist.

5. Verfahren zur Abtrennung und Reinigung von Nucleinsäure gemäß einem der Ansprüche 1 bis 4, wobei die Probenlösung, die Nucleinsäure enthält, in Schritt (1) eine Probenlösung ist, die durch Zusetzen einer Gewebelyselösung zu Tiergewebe, um das Tiergewebe einer Verdaubehandlung zu unterwerfen, hergestellt wurde.

6. Verfahren zur Abtrennung und Reinigung von Nucleinsäure gemäß einem der Ansprüche 1 bis 4, wobei die Probenlösung, die Nucleinsäure enthält, in dem vorher genannten Schritt (1) eine Probenlösung ist, die ein Antikoagulanz und wenigstens eins von Blut und weißen Blutzellen enthält.

7. Verfahren zur Abtrennung und Reinigung von Nucleinsäure gemäß Anspruch 5, wobei die Gewebelyselösung eine Lösung ist, die wenigstens eine der Verbindungen enthält, die ausgewählt sind aus der Gruppe, bestehend aus Surfactant, Puffer, Stabilisator für Nucleinsäure, Alkalimetallhalogenid, chaotropes Mittel, Protease und Antischaummittel.

8. Verfahren zur Abtrennung und Reinigung von Nucleinsäure gemäß einem der Ansprüche 1 bis 7, wobei die feste Phase, die das organische Makromolekül, das eine Polysaccharidstruktur hat, umfasst, eine feste Phase ist, die Acetylcellulose umfasst.

9. Verfahren zur Abtrennung und Reinigung von Nucleinsäure gemäß Anspruch 8, wobei die feste Phase, die Acetylcellulose umfasst, eine feste Phase ist, die ein organisches Makromolekül umfasst, das durch Unterwerfen von Acetylcellulose oder einem Gemisch von Acetylcellulosen mit verschiedenen Acetylwerten einer Verseifungsbehandlung hergestellt wurde.

10. Verfahren zur Abtrennung und Reinigung von Nucleinsäure gemäß Anspruch 9, wobei der Verseifungsgrad des Gemisches von Acetylcellulosen mit unterschiedlichen Acetylwerten 5% oder mehr ist.

11. Verfahren zur Abtrennung und Reinigung von Nucleinsäure gemäß Anspruch 1, wobei die feste Phase, die ein organisches Makromolekül umfasst, das eine Polysaccharidstruktur hat, eine feste Phase ist, die eine regenerierte Cellulose umfasst.

12. Verfahren zur Abtrennung und Reinigung von Nucleinsäure gemäß einem der Ansprüche 1 bis 11, wobei die feste Phase eine poröse Membran ist.

13. Verfahren zur Abtrennung und Reinigung von Nucleinsäure gemäß Anspruch 12, wobei die poröse Membran eine poröse Membran ist, in der die Vorder- und Rückseite asymmetrisch sind.

14. Verfahren zur Abtrennung und Reinigung von Nucleinsäure gemäß Anspruch 12, wobei die poröse Membran eine poröse Membran ist, die eine durchschnittliche Porengröße von 0,1-10,0 µm hat.

15. Verfahren zur Abtrennung und Reinigung von Nucleinsäure gemäß einem der Ansprüche 12 bis 14, wobei die poröse Membran eine poröse Membran ist, die eine Dicke von 10 bis 500 µm hat.

16. Verfahren zur Abtrennung und Reinigung von Nucleinsäure gemäß einem der Ansprüche 1 bis 15, wobei das wasserlösliche organische Lösungsmittel wenigstens eins von Methanol, Ethanol, Propanol und Butanol umfasst.

17. Verfahren zur Abtrennung und Reinigung von Nucleinsäure gemäß einem der Ansprüche 5 bis 16, das Entfernen von nichtgelösten restlichen Gewebekomponenten aus der Probelösung, die im Schritt des Zugebens einer Gewebelyselösung zu Tiergewebe, um das Tiergewebe einer Verdaubehandlung zu unterziehen, hergestellt wurde, umfasst.

18. Verfahren zur Abtrennung und Reinigung von Nucleinsäure gemäß einem der Ansprüche 5 bis 17, das Zugeben einer Lösung von RNase zu der Probenlösung, die im Schritt des Zusetzens einer Gewebelyselösung zu Tiergewebe, um Tiergewebe einer Verdaubehandlung zu unterziehen, hergestellt wurde, umfasst.

19. Verfahren zur Abtrennung und Reinigung von Nucleinsäure gemäß einem der Ansprüche 1 bis 18, wobei Adsorption und Desorption von Nucleinsäure unter Verwendung einer Einheit zur Abtrennung und Reinigung von Nucleinsäure, in der eine feste Phase in einem Behälter, der wenigstens zwei Öffnungen hat, untergebracht ist, durchgeführt werden.

20. Verfahren zur Abtrennung und Reinigung von Nucleinsäure gemäß einem der Ansprüche 1 bis 19, wobei Adsorption und Desorption von Nucleinsäure unter Verwendung einer Einheit zur Abtrennung und Reinigung von Nucleinsäure durchgeführt werden, welche (a) eine feste Phase, (b) einen Behälter, der wenigstens zwei Öffnungen hat, der die feste Phase enthält, und (c) eine Apparatur zur Erzeugung einer Druckdifferenz, die an eine der Öffnungen des vorstehend genannten Behälters angeschlossen wird, enthält.

21. Verfahren zur Abtrennung und Reinigung von Nucleinsäure gemäß Anspruch 20, wobei die Apparatur zur Erzeugung einer Druckdifferenz eine Apparatur zur Druckbeaufschlagung ist.

22. Verfahren zur Abtrennung und Reinigung von Nucleinsäure gemäß Anspruch 20, wobei die Apparatur zur Erzeugung einer Druckdifferenz eine Apparatur zur Druckverringerung ist.

23. Verfahren zur Abtrennung und Reinigung von Nucleinsäure gemäß einem der Ansprüche 20 bis 22, wobei die Apparatur zur Erzeugung einer Druckdifferenz an einer der Öffnungen des Behälters in frei lösbarer Art befestigt wird.

24. Verfahren zur Abtrennung und Reinigung von Nucleinsäure gemäß Anspruch 20 oder 21, das die folgenden Schritte umfasst:
(a) Zugeben einer Vorbehandlungslösung zu einer Probenlösung, die Nucleinsäure enthält, um eine Lösung herzustellen, die Nucleinsäure zum Adsorbieren an eine feste Phase enthält;
(b) Einspritzen einer Lösung, die Nucleinsäure zum Adsorbieren an fester Phase enthält, in eine der Öffnungen der Einheit zur Abtrennung und Reinigung von Nucleinsäure;
(c) Bringen des Inneren des Behälters in einen Druckzustand, wobei eine Apparatur zur Erzeugung einer Druckdifferenz verwendet wird, die an eine der vorstehend genannten Öffnungen der Einheit zur Abtrennung und Reinigung von Nucleinsäure befestigt ist, und Austragen einer Lösung, die Nucleinsäure zum Adsorbieren an feste Phase enthält, die injiziert wurde, aus einer anderen Öffnung, sodass die Lösung mit der festen Phase in Kontakt gebracht wird und Nucleinsäure an die feste Phase adsorbiert wird;
(d) Einspritzen einer Waschlösung in die eine der Öffnungen der Einheit zur Abtrennung und Reinigung von Nucleinsäure;
(e) Bringen des Inneren des Behälters in einen Druckzustand, wobei eine Apparatur zur Erzeugung einer Druckdifferenz, die an einer der vorstehend genannten Öffnungen der Einheit zur Abtrennung und Reinigung von Nucleinsäure befestigt ist, verwendet wird und die Waschlösung, die aus der anderen Öffnung eingespritzt wird, ausgetragen wird, sodass die Waschlösung mit der festen Phase in Kontakt gebracht wird und die feste Phase gewaschen wird;
(f) Einspritzen einer Elutionslösung, die fähig ist, die an der festen Phase adsorbierte Nucleinsäure zu desorbieren, in die andere Öffnung der Einheit zur Abtrennung und Reinigung von Nucleinsäure und
(g) Bringen des Inneren des Behälters in einen Druckzustand, wobei eine Apparatur zur Erzeugung einer Druckdifferenz, die an der einen der Öffnungen der Einheit zur Abtrennung und Reinigung von Nucleinsäure befestigt ist, verwendet wird und die Elutionslösung, die aus der anderen Öffnung eingespritzt wurde, ausgetragen wird, sodass die an der festen Phase adsorbierte Nucleinsäure desorbiert wird und zu der Außenseite des Behälters ausgetragen wird.

25. Verfahren zur Abtrennung und Reinigung von Nucleinsäure gemäß Anspruch 24, das vor dem vorher genannten Schritt (f) ein Inkontaktbringen einer DNase-Lösung mit der festen Phase und danach Waschen der festen Phase mit einer Waschlösung umfasst.

26. Verfahren zur Abtrennung und Reinigung von Nucleinsäure gemäß einem der Ansprüche 1 bis 25, wobei die Waschlösung eine Lösung ist, die 20 bis 100 Gew.-% Methanol, Ethanol, Isopropanol oder n-Propanol enthält.

27. Verfahren zur Abtrennung und Reinigung von Nucleinsäure gemäß einem der Ansprüche 1 bis 26, wobei die Elutionslösung eine Lösung ist, die eine Salzkonzentration von 0,5 mol/L oder weniger hat.

28. Reagens-Kit zur Durchführung des in einem der Ansprüche 1 bis 27 beschriebenen Verfahrens, umfassend: (i) eine Einheit zur Abtrennung und Reinigung von Nucleinsäure, (ii) Protease, (iii) ein chaotropes Salz, Surfactant und BisTris, (iv) eine Waschlösung, (v) ein Reagens für eine Elutionslösung und (vi) ein wasserlösliches organisches Lösungsmittel.

## Revendications

1. Procédé de séparation et de purification d'un acide nucléique comprenant :
(1) l'addition d'une solution de prétraitement et en outre l'addition d'un solvant organique soluble dans l'eau à une solution échantillon contenant un acide nucléique pour préparer une solution contenant un acide nucléique à adsorber sur une phase solide ;
(2) le passage de la solution contenant l'acide nucléique à adsorber sur une phase solide à travers une phase solide, pour adsorber l'acide nucléique sur la phase solide ;
(3) le passage d'une solution de lavage à travers la phase solide, pour laver la phase solide dans un état où l'acide nucléique est adsorbé ; et
(4) le passage d'une solution d'élution à travers la phase solide pour désorber l'acide nucléique de la phase solide,
dans lequel la solution de prétraitement contient du bistris(bis(2-hydroxyéthyl)iminotris(hydroxyméthyl)-méthane) en tant que tampon et est une solution contenant un tensioactif et au moins un composé choisi dans le groupe constitué par un agent anti-mousse, un stabilisant pour l'acide nucléique et un sel chaotropique, et la phase solide est une phase solide comprenant une macromolécule organique ayant la structure d'un polysaccharide.

2. Procédé de séparation et de purification d'un acide nucléique selon la revendication 1, dans lequel le tensioactif contenu dans ladite solution de prétraitement est un tensioactif non ionique.

3. Procédé de séparation et de purification d'un acide nucléique selon la revendication 2, dans lequel le tensioactif non ionique est un tensioactif du type polyoxyéthylène.

4. Procédé de séparation et de purification d'un acide nucléique selon la revendication 3, dans lequel le tensioactif du type polyoxyéthylène est un tensioactif du type polyoxyéthylène-sorbitan.

5. Procédé de séparation et de purification d'un acide nucléique selon l'une quelconque des revendications 1 à 4, dans lequel la solution échantillon contenant un acide nucléique dans l'étape (1) est une solution échantillon préparée par addition d'une solution de lyse d'un tissu à un tissu animal à soumettre à un traitement de digestion dudit tissu animal.

6. Procédé de séparation et de purification d'un acide nucléique selon l'une quelconque des revendications 1 à 4, dans lequel la solution échantillon contenant un acide nucléique dans l'étape (1) mentionnée ci-dessus est une solution échantillon contenant un anticoagulant et au moins un composé parmi le sang et les globules blancs du sang.

7. Procédé de séparation et de purification d'un acide nucléique selon la revendication 5, dans lequel la solution de lyse d'un tissu est une solution contenant l'un au moins des composés choisis dans le groupe constitué par un tensioactif, un tampon, un stabilisant pour l'acide nucléique, un halogénure de métal alcalin, un agent chaotropique, une protéase et un agent anti-mousse.

8. Procédé de séparation et de purification d'un acide nucléique selon l'une quelconque des revendications 1 à 7, dans lequel la phase solide comprenant la macromolécule organique ayant la structure d'un polysaccharide est une phase solide comprenant de l'acétylcellulose.

9. Procédé de séparation et de purification d'un acide nucléique selon la revendication 8, dans lequel la phase solide comprenant de l'acétylcellulose est une phase solide comprenant une macromolécule organique préparée par soumission d'acétylcellulose ou d'un mélange d'acétylcelluloses ayant différentes valeurs d'acétyle à un traitement de saponification.

10. Procédé de séparation et de purification d'un acide nucléique selon la revendication 9, dans lequel le taux de saponification du mélange d'acétylcelluloses ayant différentes valeurs d'acétyle est de 5 % ou plus.

11. Procédé de séparation et de purification d'un acide nucléique selon la revendication 1, dans lequel la phase solide comprenant une macromolécule organique ayant la structure d'un polysaccharide est une phase solide comprenant de la cellulose régénérée.

12. Procédé de séparation et de purification d'un acide nucléique selon l'une quelconque des revendications 1 à 11, dans lequel la phase solide est une membrane poreuse.

13. Procédé de séparation et de purification d'un acide nucléique selon la revendication 12, dans lequel la membrane poreuse est une membrane poreuse où les faces avant et arrière sont asymétriques.

14. Procédé de séparation et de purification d'un acide nucléique selon la revendication 12, dans lequel la membrane poreuse est une membrane poreuse ayant une taille moyenne de pores de 0,1 à 10,0 µm.

15. Procédé de séparation et de purification d'un acide nucléique selon l'une quelconque des revendications 12 à 14, dans lequel la membrane poreuse est une membrane poreuse ayant une épaisseur de 10 à 500 µm.

16. Procédé de séparation et de purification d'un acide nucléique selon l'une quelconque des revendications 1 à 15, dans lequel le solvant organique soluble dans l'eau comprend au moins un composé parmi le méthanol, l'éthanol, le propanol et le butanol.

17. Procédé de séparation et de purification d'un acide nucléique selon l'une quelconque des revendications 5 à 16, qui comprend l'élimination des composants tissulaires résiduels non dissous de la solution échantillon préparée dans l'étape d'addition d'une solution de lyse d'un tissu à un tissu animal pour soumettre le tissu animal à un traitement de digestion.

18. Procédé de séparation et de purification d'un acide nucléique selon l'une quelconque des revendications 5 à 17, qui comprend l'addition d'une solution de RNase à la solution échantillon préparée dans l'étape d'addition d'une solution de lyse d'un tissu à un tissu animal pour soumettre le tissu animal à un traitement de digestion.

19. Procédé de séparation et de purification d'un acide nucléique selon l'une quelconque des revendications 1 à 18, dans lequel l'adsorption et la désorption de l'acide nucléique sont réalisées à l'aide d'une unité de séparation et de purification d'un acide nucléique où une phase solide est logée dans un récipient comprenant au moins deux ouvertures.

20. Procédé de séparation et de purification d'un acide nucléique selon l'une quelconque des revendications 1 à 19, dans lequel l'adsorption et la désorption de l'acide nucléique sont réalisées à l'aide d'une unité de séparation et de purification d'un acide nucléique qui contient (a) une phase solide, (b) un récipient comprenant au moins deux ouvertures, qui loge la phase solide, et (c) un appareil de génération d'une différence de pression relié à l'une des ouvertures du récipient mentionné ci-dessus.

21. Procédé de séparation et de purification d'un acide nucléique selon la revendication 20, dans lequel l'appareil de génération d'une différence de pression est un appareil de pressurisation.

22. Procédé de séparation et de purification d'un acide nucléique selon la revendication 20, dans lequel l'appareil de génération d'une différence de pression est un appareil de dépressurisation.

23. Procédé de séparation et de purification d'un acide nucléique selon l'une quelconque des revendications 20 à 22, dans lequel l'appareil de génération d'une différence de pression est relié à l'une des ouvertures du récipient d'une manière librement détachable.

24. Procédé de séparation et de purification d'un acide nucléique selon la revendication 20 ou la revendication 21, qui comprend les étapes suivantes :
(a) l'addition d'une solution de prétraitement à une solution échantillon contenant un acide nucléique pour préparer une solution contenant un acide nucléique à adsorber sur une phase solide ;
(b) l'injection d'une solution contenant un acide nucléique à adsorber sur une phase solide dans l'une des ouvertures de l'unité de séparation et de purification d'un acide nucléique ;
(c) la mise sous pression de l'intérieur du récipient à l'aide d'un appareil de génération d'une différence de pression fixé à l'une des ouvertures mentionnées ci-dessus de l'unité de séparation et de purification d'un acide nucléique, et la décharge d'une solution contenant l'acide nucléique à adsorber sur une phase solide injectée dans celle-ci par une autre ouverture, de sorte que la solution soit mise en contact avec la phase solide et que l'acide nucléique soit adsorbé sur la phase solide ;
(d) l'injection d'une solution de lavage dans l'une des ouvertures de l'unité de séparation et de purification d'un acide nucléique ;
(e) la mise sous pression de l'intérieur du récipient à l'aide d'un appareil de génération d'une différence de pression fixé à l'une des ouvertures mentionnées ci-dessus de l'unité de séparation et de purification d'un acide nucléique, et la décharge de la solution de lavage injectée dans celle-ci par l'autre ouverture, de sorte que la solution de lavage soit mise en contact avec la phase solide et que la phase solide soit lavée ;
(f) l'injection d'une solution d'élution qui est capable de désorber l'acide nucléique adsorbé sur la phase solide dans l'autre ouverture de l'unité de séparation et de purification d'un acide nucléique ; et
(g) la mise sous pression de l'intérieur du récipient à l'aide d'un appareil de génération d'une différence de pression fixé à l'une des ouvertures de l'unité de séparation et de purification d'un acide nucléique, et la décharge de la solution d'élution injectée dans celle-ci par l'autre ouverture, de sorte que l'acide nucléique adsorbé sur la phase solide soit désorbé et déchargé à l'extérieur du récipient.

25. Procédé de séparation et de purification d'un acide nucléique selon la revendication 24 qui, avant l'étape (f) mentionnée ci-dessus, comprend la mise en contact d'une solution de DNase avec la phase solide et ensuite le lavage de la phase solide avec une solution de lavage.

26. Procédé de séparation et de purification d'un acide nucléique selon l'une quelconque des revendications 1 à 25, dans lequel la solution de lavage est une solution contenant 20 à 100 % en poids de méthanol, d'éthanol, d'isopropanol ou de n-propanol.

27. Procédé de séparation et de purification d'un acide nucléique selon l'une quelconque des revendications 1 à 26, dans lequel la solution d'élution est une solution ayant une concentration en sel de 0,5 mol/l ou moins.

28. Kit de réactifs pour réaliser le procédé mentionné dans l'une quelconque des revendications 1 à 27, comprenant : (i) une unité de séparation et de purification d'un acide nucléique, (ii) une protéase, (iii) un sel chaotropique, un tensioactif et du bistris, (iv) une solution de lavage, (v) un réactif pour une solution d'élution et (vi) un solvant organique soluble dans l'eau.
